(19) Europäisches Patentamt
European Patent Office
Office européen des brevets

(11) **EP 2 793 693 B1**

(12) **EUROPÄISCHE PATENTSCHRIFT**

(45) Veröffentlichungstag und Bekanntmachung des Hinweises auf die Patenterteilung:
**24.02.2016 Patentblatt 2016/08**

(21) Anmeldenummer: **12818516.2**

(22) Anmeldetag: **21.12.2012**

(51) Int Cl.:
*A61B 5/04* *(2006.01)*　　*A61B 5/048* *(2006.01)*
*A61B 5/00* *(2006.01)*

(86) Internationale Anmeldenummer:
**PCT/EP2012/076854**

(87) Internationale Veröffentlichungsnummer:
**WO 2013/093105 (27.06.2013 Gazette 2013/26)**

(54) **VORRICHTUNG UND SYSTEM ZUR BESTIMMUNG VON GEHIRNAKTIVITÄTEN**

DEVICE AND SYSTEM FOR DETERMINING BRAIN ACTIVITIES

PROCÉDÉ ET SYSTÈME SERVANT À DÉTERMINER LES ACTIVITÉS CÉRÉBRALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 DE 102011122025**

(43) Veröffentlichungstag der Anmeldung:
**29.10.2014 Patentblatt 2014/44**

(73) Patentinhaber: **Hipp, Jörg Felix**
**79539 Lörrach (DE)**

(72) Erfinder: **Hipp, Jörg Felix**
**79539 Lörrach (DE)**

(74) Vertreter: **UEXKÜLL & STOLBERG**
**Patentanwälte**
**Beselerstrasse 4**
**22607 Hamburg (DE)**

(56) Entgegenhaltungen:
**US-A1- 2001 049 480　　US-A1- 2011 004 115**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung bezieht sich auf ein Verfahren zur Bestimmung eines Maßes für einen Zusammenhang zwischen einer mit einem ersten Sensor gemessenen elektrischen oder magnetischen Aktivität des Gehirns und einer mit einem zweiten Sensor gemessenen elektrischen oder magnetischen Aktivität des Gehirns sowie ein System zur Durchführung des Verfahrens.

Für verschiedene Anwendungen besteht der Wunsch, die Funktion eines menschlichen oder tierischen Gehirns messtechnisch erfassen zu können. Entsprechende Messungen erfassen dabei üblicherweise die Aktivität des Gehirns, die auf verschiedene Weisen gemessen werden kann. Der Begriff der Aktivität des Gehirns ist weit gefasst. Man kann darunter bereits allgemein physiologische Vorgänge in einzelnen Gehirnzellen verstehen, die zur Verarbeitung von Informationen ihren elektrischen Zustand verändern. Wenn nicht nur eine einzelne Zelle ihren elektrischen Zustand verändert, sondern ganze Bereiche des Gehirns, so entstehen großflächige Potentialschwankungen, die sich durch Volumenleitung über das gesamte Gehirn ausbreiten. Die Ursprünge dieser großflächigen Potentialschwankungen werden im Folgenden als Quellen bezeichnet. Unter einer Teilaktivität soll im Folgenden der Anteil der an einem Sensor gemessenen Aktivität verstanden werden, der von einer bestimmten Quelle erzeugt wird. Bringt man geeignete Sensoren bzw. Elektroden auf der Kopfhaut an, so kann man die elektrische Aktivität des Gehirns als die Summe dieser Potential- oder auch Spannungsschwankungen am Ort der jeweiligen Sensoren relativ zu einer Referenz direkt messen. In diesem Sinne ist der Begriff der elektrischen Aktivität hier zu verstehen. Dieses Messverfahren wird als Elektroenzephalografie (EEG) bezeichnet. Alternativ kann man mit geeigneten Sensoren anstelle der elektrischen Potentialschwankungen auch die gleichzeitig auftretenden magnetischen Felder messen, die hier als die magnetische Aktivität des Gehirns verstanden werden. Man spricht in diesem Fall von Magnetoenzephalographie (MEG). Die magnetischen Felder weisen gegenüber den elektrischen Feldern deutliche geringere Effektgrößen auf. Entsprechend aufwändiger müssen die Sensoren und deren Abschirmung von Störsignalen aus der Umgebung ausgestaltet sein. Davon abgesehen, weisen EEG und MEG vergleichbare Eigenschaften auf. Insbesondere können die Signale in beiden Fällen mit einer zeitlichen Auflösung vermessen werden, die besser als 1 ms ist.

Da die zeitliche Variationen der Potentialschwankungen relativ tieffrequent sind und typischerweise weniger als 1000 Hz betragen, und kapazitive sowie induktive Eigenschaften des Gewebes in diesem Frequenzbereich in erster Näherung vernachlässigbar sind, werden Spannungsschwankung bzw. Magnetfeldschwankung quasi gleichzeitig an allen Sensoren gemessen. Wird die von einer Quelle ausgehende Teilaktivität an zwei beabstandet angeordneten Sensoren gemessen, so weisen die von den Sensoren ausgegebenen Signale somit abhängig von dem Abstand zur Quelle und deren Orientierung zwar unterschiedliche Amplituden jedoch keinen Phasenunterschied auf. In diesem Sinne besteht eine instantane Beziehung zwischen den von den beiden Sensoren ausgegeben Signalen. Eine räumliche Zuordnung der Aktivität zu einer bestimmten Region des Gehirns bzw. einer bestimmten Quelle ist daher nur über die relative gemessene Signalintensität an verschiedenen Sensoren möglich, die abhängig von der Orientierung der Quelle ist und mit dem Abstand zwischen dem jeweiligen Sensor und der Quelle abnimmt. So ist es insbesondere nicht möglich die Signale von einzelnen Sensoren eindeutig bestimmten Quellen zuzuordnen. Bei entsprechender Orientierung können Quellen nämlich noch am anderen Ende des Kopfes gemessen werden.

Es ist im Prinzip möglich, bei einer gleichzeitigen Messung mit einer relativ großen Anzahl von Sensoren eine Quellenlokalisation durchzuführen. Da jedoch von einer oberflächlichen Messung auf eine räumliche Quellenverteilung geschlossen werden muss, ist die Lösung im Allgemeinen nicht eindeutig. Im Allgemeinen existieren zu jedem gemessen elektrischen bzw. magnetischen Feld unendlich viele Möglichkeiten an Quellenkonfigurationen, die die Messung erklären könnten. Nur wenn im Voraus das Problem eingegrenzt werden kann, z.B. wenn eine feste Anzahl Quellen, die Auslöser einer Aktivität sein können, angenommen werden kann oder die genaue räumlich Verteilung der Quellen bekannt ist, so kann bei hinreichend genauer Modellierung eindeutig auf die Lage und Stärke einer eine bestimmte Teilaktivität erzeugenden Quelle geschlossen werden. In der Regel sind die so gefundenen Lösungen jedoch nicht eindeutig. Zudem sind zur Lösung des inversen Problems in der Regel eine anatomische Aufnahme des Gehirns und die Erstellung eines Vorwärtsmodells des Kopfes notwendig. Im Allgemeinen liegt die Genauigkeit der Ortsbestimmung nur in der Größenordnung von einigen Zentimetern und stellt ein räumlich verschmiertes und fehlerhaftes Abbild der wahren Quellen dar. Methoden zur Quellenlokalisation können somit die räumliche Spezifizität erhöhen, stellen jedoch keine Möglichkeit dar, die Aktivitäten verschiedener Quellen zuverlässig zu trennen.

Normale Gehirnfunktionen bestehen in der Regel aus einer Vielzahl von parallel ablaufenden Teilaktivitäten. Diese tauschen - ähnlich wie bei einem Kommunikationsnetzwerk - untereinander Informationen aus. Die einfache Messung der Intensität einer oder mehrerer Teilaktivitäten ermöglicht es daher oft nicht, ein vollständiges Bild über die Gehirnfunktion zu erlangen. Um eine besseres Verständnis der Zusammenhänge der im Gehirn ablaufenden Teilaktivitäten zu erhalten, wäre es vielmehr wünschenswert, wenn man Kenntnisse über die Kommunikation bzw. den Zusammenhang der verschiedenen Teilaktivitäten des Gehirns erhalten würde.

Hierbei ergibt sich jedoch eine Vielzahl von Problemen. Sind zwei oder mehr Quellen gleichzeitig aktiv, so überlagern sich deren elektrischen bzw. magnetischen Teilaktivitäten an den Sensoren, und die Sensoren erfassen eine Mischung oder mit anderen Worten eine Linearkombination der Teilaktivitäten. Es ist dann meist nicht mehr möglich, aus den Signalintensitäten der von den verschiedenen Sensoren ausgegeben Signale ein Maß für neuronale Interaktionen bzw. Zusammenhänge zu bestimmen, da die Sensoren in aller Regel in jeweils unbekanntem Umfang die von denselben Quellen erzeugten Teilaktivitäten erfassen. Einzig wenn die Teilaktivitäten bzw. deren Quellen sehr unterschiedliche und bekannte Eigenschaften haben, z.B. wenn die Aktivitäten in unterschiedlichen Frequenzbändern liegen, lassen sich die von den Sensoren ausgegebenen Signale beispielsweise durch die Anwendung von Bandpassfiltern den jeweiligen Aktivitäten des Gehirns zuordnen. Dann lässt sich daraus im Prinzip auch ableiten, ob zwei Aktivitäten, die in unterschiedlichen Frequenzbändern liegen, zusammenhängen.

Oftmals laufen aber gerade kausal zusammenhängende Aktivitäten im gleichen Frequenzband oder zumindest in überlappenden Frequenzbändern ab, so dass es von besonderem Interesse ist, den Zusammenhang derartiger Aktivitäten zu untersuchen. Eine Auftrennung und Überprüfung der ausgegebenen Signale auf einen Zusammenhang hin ist daher technisch höchst problematisch.

Insbesondere ist es bisher nicht zuverlässig und in einfacher Weise möglich einen Zusammenhang basierend auf den sich zeitlich verändernden Signalen verschiedener Quellen zu bestimmen. Versucht man nämlich einen Zusammenhang der von den Sensoren ausgegebenen Signale zu berechnen, zum Beispiel in Form einer Korrelation der Signale oder zeitabhängiger Maße, die die Signale beschreiben, so steht man vor dem oben bereits angedeuteten Problem, dass mehrere Sensoren in der Regel zu einem von ihrer Position abhängigen aber unbekannten Anteil die gleichen Teilaktivitäten messen. Stellt man einen Zusammenhang zweier von zwei Sensoren gelieferten Signalen fest, so kann es sich somit entweder um den festzustellenden Zusammenhang zweier unterschiedlicher Aktivitäten handeln, oder aber lediglich um eine von der gleichen Quelle erzeugte Teilaktivität, deren elektromagnetischen Felder an den zwei Sensoren gleichzeitig gemessen wurden.

Aus Nolte et al., "Identifying true brain interaction from EEG data using the imaginary part of coherency", Clinical Neurophysiology, 2004, Band 115, Seiten 2292 bis 2307, im Folgenden als "Verfahren der imaginären Kohärenz" bezeichnet, ist ein Verfahren zur Bestimmung eines Zusammenhangs zwischen den an zwei Sensoren gemessenen Aktivitäten des Gehirns bekannt. Bei dem dort beschrieben Verfahren wird ein Zusammenhang zwischen den von den beiden Sensoren ausgegebenen Signalen berechnet, wobei als Maß für den Zusammenhang die Kohärenz verwendet wird, die systematische Beziehungen auf kleinster zeitlicher Skala erfasst. Um vermeintliche Zusammenhänge auszuschließen, die lediglich durch Messung der gleichen Teilaktivitäten an beiden Sensoren entstehen, wird nur der imaginäre Anteil der berechneten Kohärenz berücksichtigt. Dieser Anteil ist jedoch schwierig zu interpretieren, da er stark von der Phasenverschiebung zwischen den beiden Signalen abhängt, die sich durch verschiedene Einflüsse mit der Zeit ändern kann. Eine Veränderung des imaginären Anteils kann also auf die Veränderung eines Zusammenhangs zwischen zwei ausgegebenen Signalen hindeuten oder lediglich durch eine Veränderung in der Phasenverschiebung bedingt sein. Es ist nicht möglich, zwischen diesen beiden Effekten zu unterscheiden. Aus diesem Grund besteht die Gefahr, dass tatsächlich existierende Zusammenhänge verdeckt werden. Außerdem ist es relativ schwierig, die für die Bestimmung der Kohärenz notwendige Phaseninformation der neuronalen Aktivität zu messen. Abgesehen von dem Problem, Phasenbeziehungen robust zu erfassen können zwei Hirnaktivitäten miteinander in Beziehung stehen, ohne dass diese sich auf der feinsten zeitlichen Skala, d.h. in Form einer systematischen Phasenbeziehung, widerspiegeln muss. Die Methode der imaginären Kohärenz kann diese Beziehungen nicht erfassen.

Eine deutliche höhere räumliche Spezifizität bietet das aus dem EEG abgeleitete Elektrocorticogramm (ECoG), bei dem die Sensoren durch die chirurgisch geöffnete Schädeldecke direkt auf die zu untersuchende Hirnrinde aufgelegt oder in sie eingeführt werden. Beim ECoG lassen sich räumliche Auflösungen von weniger als einem Zentimeter erreichen. Prinzipiell ergibt sich jedoch die gleiche Problematik wie beim EEG, das auf der Kopfhaut gemessen wird. Dieses Verfahren wird ferner nur zur Vorbereitung von schwerwiegenden chirurgischen Eingriffen z.B. bei Epilepsie eingesetzt, da die mit einem derartigen invasiven Eingriff verbundenen Risiken nur in solchen Ausnahmefällen vertretbar sind.

Eine weitere nicht-invasive Methode zur Messung der Hirnaktivität ist die funktionelle Magnetresonanztomographie (fMRT). Insbesondere unter Ausnutzung des sog. BOLD-Kontrastes (Blood Oxygen Level Dependent-Kontrast) ist es möglich, Durchblutungsänderungen von Hirnarealen sichtbar zu machen. Bei einer Aktivierung einer Gehirnregion kommt es dort zu einer deutlichen Erhöhung des Sauerstoffgehaltes des Blutes, die sich in einem veränderten BOLD-Kontrast wiederspiegelt. Die Auflösung von so gewonnenen fMRT-Aufnahmen ist kleiner als 1 cm. Die Änderung des BOLD Signals das mit der Aktivität verschiedener Hirnregionen einhergeht lässt sich also räumlich gut trennen. Allerdings ist die zeitliche Auflösung der fMRT deutlich schlechter als die von EEG- oder MEG-Messungen. fMRT-Bilder werden in der Regel alle 2 bis 4 Sekunden aufgezeichnet. Zudem tritt die Veränderung des BOLD-Kontrastes mit einigen Sekunden Verzögerung ein. Das maximale Signal wird ungefähr 6 Sekunden nach dem Beginn der Aktivität in einer Gehirnregion beobachtet und kann zwischen unterschiedlichen Gehirnregionen deutlich variieren. Es ist daher zwar möglich einen über die Sauerstoffkonzentration indirekt bestimmten Zusammenhang zweier räumlich beschränkter Aktivitäten auf langen Zeitskalen festzustellen, allerdings kann die Methode nicht die Kommunikation zwischen zwei Aktivitäten auf schnelleren Zeitskalen erfassen und in Zusammenhang setzen.

Andere funktionelle Bildgebungsmethoden umfassen die Positronenemissionstomographie (PET) und die Single-Photon Emission Computed Tomography (SPECT). Diese Methoden verwenden radioaktive Tracer, die sich beispielsweise dort ansammeln, wo besonders viel Glukose umgesetzt wird. Die räumliche Auflösung dieser Methoden ist kleiner als 1 cm, allerdings dauert die Aufnahme eines vollständigen 3D-Datensatzes oftmals mehr als eine Stunde, so dass nur langfristig aktive Gehirnregionen von langfristig inaktiven Gehirnregionen unterschieden werden können. Zur Messung des Zusammenhangs der Aktivität verschiedener Gehirnregionen ist diese Methode daher ungeeignet. Zudem ist die Verwendung von radioaktiven Tracern mit einer nicht unerheblichen, nachteiligen Strahlenbelastung des untersuchten Menschen verbunden.

Neben dem hohen finanziellen und - insbesondere bei der PET - technischen Aufwand, den sämtliche funktionellen Bildgebungsmethoden mit sich bringen, liegt ihr Hauptnachteil jedoch darin, dass sie nie die eigentliche Aktivität des Gehirnes messen, sondern stets die Intensität eines Surrogates. Das kann im Fall der MRT unter Ausnutzung des BOLD-Kontrastes der Sauerstoffgehalt des Blutes sein oder im Fall der PET der Glukoseverbrauch. Eine Veränderung der Intensität eines dieser Surrogate kann jedoch immer auch eine andere Ursache als eine gesteigerte Gehirnaktivität haben. So wird insbesondere die PET mit 18F-Fluordesoxyglucose- (FDG-) Tracern zur Lokalisation von Tumoren im Gehirn verwendet, die einen dauerhaft erhöhten Glukoseumsatz aufweisen. Daher sind die Ergebnisse dieser Messverfahren mit Vorsicht zu betrachten. Auch haben verschiedene Gehirnaktivitäten mit unterschiedlichen charakteristischen elektrophysiologischen Signaturen (z.B. oszillatorische Prozesse mit einer charakteristischen Zeitkonstante) eine nicht eindeutige oder unbekannte Relation zum Sauerstoffverbrauch bzw. zu dem entsprechenden Surrogat und können somit mit funktionellen Bildgebungsmethoden nicht oder nur stark eingeschränkt untersucht werden. Schließlich lassen sich Reaktions- und Denkprozesse, die in Zeiträumen deutlich unter einer Sekunde ablaufen, mit diesen Verfahren nur im zeitlichen Mittel darstellen, wodurch eventuell vorhandene kausale Zusammenhänge verdeckt werden.

Ein weiteres System und Verfahren zur Bestimmung eines Zusammenhangs zwischen den von mehreren Sensoren gemessenen Aktivitäten des Gehirns wird in US 2001/0049480A1 vorgestellt. Mit diesem System werden dem Nutzer mehrere Stimuli präsentiert, die derart ausgebildet sein müssen, dass die Frequenzbereiche der gemessenen Reaktionen auf die jeweiligen Stimuli nicht miteinander interferieren. Somit ist es möglich, eine klare Beziehung zwischen dem jeweiligen Stimulus und dessen zugehöriger Reaktion herzustellen.

Es ist Aufgabe der Erfindung, ein einfach und schnell durchzuführendes Verfahren und ein einfach aufgebautes und zu handhabendes System anzugeben, mit denen ein Maß für einen Zusammenhang zwischen einer mit einem ersten Sensor gemessenen ersten elektrischen oder magnetischen Aktivität des Gehirns und einer mit einem zweiten Sensor gemessenen zweiten elektrischen oder magnetischen Aktivität des Gehirns zuverlässig bestimmt werden kann und die Nachteile des Standes der Technik vermieden werden. Insbesondere soll gegenüber dem Stand der Technik eine verbesserte zeitliche Auflösung erreicht werden, nicht-invasiv vorgegangen werden und der bestimmte Zusammenhang robust sein, insbesondere gegenüber Veränderungen der Phasenverschiebung zwischen den ausgegebenen Signalen.

Diese Aufgabe wird durch ein Verfahren mit den Merkmalen von Patentanspruch 1 und ein System mit den Merkmalen des Anspruchs 15 gelöst. Vorteilhafte Ausführungsformen des Verfahrens und des Systems sind Gegenstand der jeweils zugehörigen Unteransprüche.

Das Verfahren ist zur Bestimmung eines Maßes für einen Zusammenhang, wie zum Beispiel eine Korrelation, zwischen verschiedenen Aktivitäten des Gehirns und beispielsweise den Aktivitäten räumlich getrennter Gehirnbereiche - d.h. die Interaktion der Aktivitäten des Gehirns und beispielsweise den Aktivitäten verschiedener Hirnbereiche - ausgestaltet.

Dazu werden ein mit Hilfe eines ersten Sensors erzeugtes oder von einem ersten Sensor ausgegebenes erstes zeitabhängiges Signal und ein mit Hilfe eines zweiten Sensors erzeugtes oder von einem zweiten Sensor ausgegebenes zweites zeitabhängiges Signal zeitgleich aufgezeichnet. Der erste Sensor und der zweite Sensor messen jeweils eine erste bzw. zweite magnetische oder elektrische Aktivität des Gehirns, wobei es bevorzugt ist, wenn entweder beide Sensoren eine elektrische Aktivität messen oder beide Sensoren eine magnetische Aktivität messen. Das erste zeitabhängige Signal ist für die gemessene erste elektrische oder magnetische Aktivität charakteristisch, und das zweite zeitabhängige Signal ist für die gemessene zweite elektrische oder magnetische Aktivität charakteristisch. Der erste Sensor und der zweite Sensor sind voneinander beabstandet angeordnet, d.h. die beiden Sensoren werden in einer räumlichen Entfernung voneinander angeordnet, bevorzugt an einem Kopf oder in der Nähe eines Kopfes. Unter Aktivität soll dabei hier die Summe der am Ort des jeweiligen Sensors messbaren im Gehirn erzeugten elektrischen Potentialschwankungen oder magnetischen Feldschwankungen verstanden werden. Die Ursprünge dieser großflächigen Potential- bzw. Feldschwankungen werden im Folgenden als Quellen bezeichnet, der von ihnen erzeugte Anteil einer Aktivität als Teilaktivität. Es ist zu beachten, dass die Verwendung des Ausdrucks Quelle in keiner Weise dahingehend einschränkend zu verstehen ist, dass die Ursprünge der Aktivitäten zwingend auf einen beschränkten Ort festlegbar sind. Es kann sich durchaus auch um großflächige Aktivitäten handeln, in denen mehrere Areale des Gehirns aktiv sind, und Quellen verschiedener Teilaktivitäten können sich räumlich überlappen. In der Regel wird eine Vielzahl von Quellen zu der an einem Sensor gemessenen Aktivität beitragen. Zudem beeinflusst die gleiche Quelle regelmäßig sowohl die am ersten Sensor als auch die am zweiten Sensor gemessenen Aktivität. Die Aufzeichnung der Signale erfolgt vorzugsweise mittels einer Datenverarbeitungseinheit, z.B. auf einem Datenträger.

In einer bevorzugten Ausführungsform werden elektrische Aktivitäten mittels Elektroenzephalografie (EEG) und magnetische Aktivitäten mittels Magneteoenzephalografie (MEG) gemessen. Entsprechend handelt es sich bei den Sensoren um z.B. entweder auf der Kopfhaut aufgebrachte Elektroden oder um den Kopf herum angeordnete Magnetfeldsensoren, die beispielsweise durch supraleitende Spulen oder auch sogenannte SQUID Sensoren gebildet werden können. Es ist auch vorstellbar, die Aktivitäten mittels Elektrocorticogramm- (ECoG-) Sensoren aufzuzeichnen, die beispielsweise direkt auf oder in der Hirnrinde angeordnet sind.

Es ist bevorzugt, dass die Sensoren so ausreichend beabstandet zueinander angeordnet werden, dass die Überlagerung der von verschiedenen Quellen hervorgerufenen elektrischen bzw. magnetischen Felder zu unterschiedlichen Aktivitäten am ersten und am zweiten Sensor führt. Der gewählte Abstand hängt dabei von den zu untersuchenden Aktivitäten ab, sollte aber typischerweise bevorzugt einige Zentimeter betragen, zum Beispiel mindestens 1 Zentimeter, bevorzugt mindestens 3 Zentimeter, mehr bevorzugt mindestens 4 Zentimeter und am meisten bevorzugt mindestens 5 Zentimeter. Insgesamt sollte der Abstand so gewählt werden, dass die beiden Signale ausreichend unterschiedlich sind, damit sie sich bei der im Folgenden beschriebenen Signalverarbeitung nicht im Wesentlichen vollständig auslöschen. Die Positionen der Sensoren am Kopf oder in der Nähe des Kopfes werden jeweils durch die bei einer bestimmten Anwendung zu beobachtenden Aktivitäten bestimmt.

Das erste und das zweite Signal können entweder direkt von dem jeweiligen Sensor bzw. der jeweiligen Sensorelektronik geliefert werden oder sie können sich nach einer optionalen Vorverarbeitung der eigentlichen Sensorsignale ergeben. Beispielsweise kann ein Sensorsignal von Artefakten bereinigt werden.

Eine solche Vorverarbeitung kann in einer bevorzugten Ausführungsform für einen oder beide Sensoren eine geeignete Kombination der von einer jeweiligen Vielzahl von Sensorelementen ausgegebenen Signale zu einem kombinierten Signal bzw. dem entsprechenden ersten bzw. zweiten Signal umfassen. Mit anderen Worten kann einer der Sensoren oder können beide Sensoren eine Vielzahl von separaten Sensorelementen oder Einzelsensoren aufweisen, wobei das jeweilige zeitabhängige Signal aus einer Linearkombination der mit Hilfe der Einzelsensoren erzeugten bzw. von den Einzelsensoren ausgegebenen Signale gebildet wird. Die Einzelsensoren können z.B. räumlich beabstandet voneinander angeordnet sein. Es ist dabei auch vorstellbar, dass ein oder mehrere Einzelsensoren sowohl dem ersten Sensor als auch dem zweiten Sensor zugeordnet wird bzw. werden. Beispielsweise könnten im Extremfall der erste Sensor und der zweite Sensor dieselben Einzelsensoren aufweisen. Das erste Signal und das zweite Signal unterscheiden sich in diesem Fall z.B. lediglich dadurch, dass sie mittels unterschiedlicher Linearkombinationen aus den mit Hilfe der Einzelsensoren erzeugten bzw. von den Einzelsensoren ausgegebenen Signalen gebildet werden.

Es kann von Vorteil sein, wenn derartige Sensoren, die mehrere Sensorelemente bzw. Einzelsensoren aufweisen, zum Einsatz kommen und eine Quellenlokalisation durchgeführt wird. In diesem Fall könnte es sich bei dem ersten Signal und dem zweiten Signal auch um die mittels Quellenlokalisation einer ersten und einer zweiten Quelle zügeordneten Signale handeln. Eine Quellenlokalisation durchzuführen ist besonders vorteilhaft, da sich in diesem Falle mit dem erfindungsgemäßen Verfahren Aussagen über den Zusammenhang der Aktivitäten der räumlich lokalisierten Quellen machen lassen.

In jedem Fall werden bei dem erfindungsgemäßen Verfahren ferner ein oder mehrere Frequenzbereiche des ersten Signals und des zweiten Signals ausgewählt. Der Frequenzbereich bzw. die Frequenzbereiche sind für die beiden Signale identisch. Die Auswahl des Frequenzbereiches bzw. der Frequenzbereiche hängt dabei davon ab, welche Aktivitäten im Hirn beobachtet werden sollen. Beispielsweise kann für eine Untersuchung der sogenannte Alpha-Frequenzbereich, der zwischen 8 und 12 Hz liegt, ausgewählt werden. Untersuchungen in diesem Frequenzbereich könnten zum Beispiel Aufschluss über den Wachzustand eines Patienten liefern oder über pathologische Veränderungen von Kommunikationsprozessen. Es ist im Extremfall auch denkbar, dass nur ein Frequenzbereich ausgewählt wird, der alle in den aufgezeichneten Signalen auftretenden Frequenzen umfasst.

Ferner wird für jeden Frequenzbereich ein Zusammenhang zwischen dem ersten Signal und dem zweiten Signal bestimmt bzw. das Fehlen eines Zusammenhangs festgestellt. Dazu wird zunächst für jeden Frequenzbereich ein erstes frequenzbeschränktes Signal aus dem ersten Signal und ein zweiten frequenzbeschränkten Signal aus dem zweiten Signal ausgewählt bzw. gebildet. Die Frequenzbeschränkung bezieht sich dabei auf den jeweiligen Frequenzbereich und kann beispielsweise stattfinden, indem auf das erste Signal und auf das zweite Signal derselbe, dem Frequenzbereich entsprechende bzw. vom Frequenzbereich abhängige Bandpassfilter angewendet wird.

In einem weiteren Schritt wird für jeden Frequenzbereich eine erste Einhüllende des ersten frequenzbeschränkten Signals gebildet. Die Einhüllende kann z.B. bevorzugt die Signalenergie angeben. Die Einhüllende kann somit beispielsweise berechnet werden, indem der Logarithmus vom Quadrat des Absolutbetrages des Signals bzw. des jeweiligen Anteils des Signals berechnet wird. Auf diese Weise wird die Verteilung der Signalenergien näher an eine Normalverteilung gerückt, wodurch eine nachfolgende Berechnung eines Maßes für den Zusammenhang erleichtert wird.

Außerdem wird für jeden Frequenzbereich eine zweite Einhüllenden eines Anteils des zweiten frequenzbeschränkten Signals bestimmt, wobei der Anteil eine geringere lineare Korrelation mit dem ersten frequenzbeschränkten Signal aufweist als das zweite frequenzbeschränkte Signal. Der Anteil des zweiten frequenzbeschränkten Signals weist damit nur noch in geringerem Umfang als das zweite frequenzbeschränkte Signal selbst Beiträge von solchen Teilaktivitäten auf, die sowohl am ersten als auch am zweiten Sensor gemessen werden.

Mit anderen Worten ist die zweite Einhüllende die Einhüllende eines Anteils des zweiten frequenzbeschränkten Signals, der sich nicht durch eine Skalierung des ersten frequenzbeschränkten Signals mit einem reellen Faktor bestimmen lässt. Der Faktor wird so gewählt, dass die lineare Korrelation zwischen dem ersten und dem zweiten frequenzbeschränkten Signal größer ist als die lineare Korrelation zwischen dem ersten frequenzbeschränkten Signal und dem Anteil des zweiten frequenzbeschränkten Signals.

Zum besseren Verständnis dieses Schrittes ist noch einmal darauf hinzuweisen, dass bei der Messung von Aktivitäten des Gehirns mit zwei Sensoren zu einem unbekannten Anteil die gleichen Teilaktivitäten an beiden Sensoren mit unterschiedlicher Amplitude aber gleicher Phase gemessen werden, da sich, wie oben erläutert, elektrische bzw. magnetische Felder im Kopf quasi instantan ausbreiten. Das Verhältnis der Intensitäten, mit denen dieselbe Teilaktivität am ersten und zweiten Sensor gemessen wird, bleibt jedoch konstant, solange sich die Position der Sensoren in Bezug auf die Quellen dieser Teilaktivitäten nicht verändert. Der Anteil des zweiten frequenzbeschränkten Signals, der der an beiden Sensoren gemessenen Teilaktivitäten entspricht, steht somit in einem festen Verhältnis zu dem Anteil des ersten frequenzbeschränkten Signals, der der an beiden Sensoren gemessenen Teilaktivitäten entspricht. Alleine die Messung der gleichen Teilaktivität an beiden Sensoren führt also dazu, dass eine lineare Korrelation zwischen dem ersten und dem zweiten frequenzbeschränkten Signal besteht. Ziel des vorliegenden Schrittes ist es, einen Anteil des zweiten frequenzbeschränkten Signals zu bestimmen, der diese lineare Korrelation nur zu einem geringeren Grad aufweist, als das zweite frequenzbeschränkte Signal und im Idealfall eine lineare Korrelation von Null oder im Wesentlichen Null.

Dem Fachmann ist eine Vielzahl von Verfahren bekannt, mit denen eine lineare Korrelation zwischen dem ersten und dem Anteil des zweiten frequenzbeschränkten Signal bestimmt werden kann. Die Auswahl eines geeigneten Verfahrens hängt dabei insbesondere von dem Zeitbereich ab, in dem die Beeinflussung des ersten und des zweiten frequenzbeschränkten Signals durch die gleichen Teilaktivitäten vorliegt, und ob die Bestimmung des Zusammenhangs zwischen dem ersten und dem zweiten frequenzbeschränkten Signal in der Zeit- oder der Frequenzdomäne erfolgt. Einige beispielhafte Verfahren werden in den folgenden bevorzugten Ausführungsbeispielen dargestellt, wobei hervorzuheben ist, dass die Darstellung expliziter mathematischer Methoden vornehmlich der Illustration des dahinterstehenden Grundprinzips dient.

Durch die Bestimmung der zweiten Einhüllenden als Einhüllende des Anteils des zweiten frequenzbeschränkten Signals wird sichergestellt, dass zwischen der ersten Einhüllenden und der zweiten Einhüllenden im weiteren Verlauf des erfindungsgemäßen Verfahrens ein vermeintlicher Zusammenhang bzw. eine vermeintliche lineare Korrelation nicht oder nur in vermindertem Umfang bestimmt wird, die lediglich darauf beruht, dass die von einer Quelle erzeugte Teilaktivität ohne jeden Zeitverzug, d.h., instantan an beiden Sensoren gemessen wird. Mit anderen Worten wird bevorzugt die Einhüllende von dem Anteil des zweiten Signals gebildet, der möglichst wenige und im Idealfall keine oder im Wesentlichen keine Teilaktivitäten berücksichtigt, die in anderer Linearkombination zeitgleich d.h. instantan auch am ersten Sensor gemessen wurden.

Schließlich wird für jeden Frequenzbereich ein Maß für den Zusammenhang, wie zum Beispiel eine Korrelation, zwischen der ersten Einhüllenden und der zweiten Einhüllenden als Maß für einen Zusammenhang zwischen der mit dem ersten Sensor gemessenen elektrischen oder magnetischen Aktivität des Gehirns und der mit dem zweiten Sensor gemessenen elektrischen oder magnetischen Aktivität des Gehirns bestimmt. Ein Maß für den Zusammenhang kann beispielsweise auf Grund der Linearen Korrelation, der Rangkorrelation, der Transinformation, der Granger-Kausalität oder anderer Korrelationsmaße ermittelt werden.

Das oben beschriebene Verfahren hat zunächst den Vorteil, dass ein Zusammenhang zwischen zwei Einhüllenden bestimmt wird und nicht zwischen den beiden unmittelbar durch die beiden Sensoren gelieferten Signale. Ferner hat das Verfahren den Vorteil, dass der tatsächliche Zusammenhang bzw. die tatsächliche Korrelation der Einhüllenden zweier gemessener Aktivitäten bestimmt werden kann, von denen jede nicht zwangsläufig einer separaten Quelle entsprechen muss, sondern auch eine Kombination mehrerer, ggf. nicht lokalisierter Quellen sein kann. Im Gegensatz zum Stand der Technik können damit in einer einfachen, robusten und zuverlässigen Weise nicht nur ggf. Aussagen über die Stärke von Aktivitäten sondern insbesondere über die Kommunikation zwischen verteilten Hirnprozessen gemacht werden. Bisher existierenden EEG- oder MEG-Verfahren ist es überlegen, da die gleichzeitige Messung einer Quelle an mehreren Sensoren nicht zur fälschlichen Feststellung eines Zusammenhangs führt. Dadurch, dass der Zusammenhang zwischen der Einhüllenden des ersten Signals und der Einhüllenden eines Anteils des zweiten Signals berechnet wird, werden die Anteile, die aus den gleichen Quellen stammen, nicht oder zumindest nur in verringertem Maße berücksichtigt. Diese Problematik tritt bei funktionellen Bildgebungsverfahren zwar nicht auf, allerdings ist es diesen Verfahren deutlich in Bezug auf seine zeitliche und spektrale Auflösung sowie in Bezug auf die Einfachheit der Durchführung und des Aufbaus überlegen. Drüber hinaus erfassen EEG und MEG direkt die neuronale Aktivität und kein Surrogat wie der Sauerstoffsättigung im Blut oder den Glukoseverbrauch, die auch durch andere, nicht neuronale Faktoren moduliert werden können.

Es ist darauf hinzuweisen, dass sich das beanspruchte Verfahren von dem Verfahren der imaginären Kohärenz in mehreren wesentlichen Aspekten unterscheidet. Zunächst einmal wird bei dem erfindungsgemäßen Verfahren ein Zusammenhang zwischen zwei Einhüllenden und nicht zwischen den gemessenen Signalen selbst bestimmt. Die Maße beschreiben also verschiedene Vorgänge, die auf unterschiedlichen Zeitskalen stattfinden. Die Bestimmung des Zusammenhangs zweier Einhüllender ist unempfindlich gegenüber Vorgängen auf der feinsten zeitlichen Skala wie z.B. der Phasenverschiebungen zwischen den beiden Signalen, da sich die Werte von Einhüllenden deutlich langsamer ändern als die der Signale selbst. Das hat zur Folge, dass das erfindungsgemäße Verfahren im Gegensatz zu dem Verfahren der imaginären Kohärenz in vorteilhafter Weise robust gegenüber den nicht interessierenden Einflüssen ist, die zu Phasenverschiebungen der eigentlichen Signale führen können.

Darüber hinaus ist davon auszugehen, dass Phasensynchronisation eine schwerer zu messende und seltener auftretende Signatur von Kommunikationsprozessen ist. Mit anderen Worten ist davon auszugehen, dass viele Kommunikationsprozesse sich durch Korrelation der Signalenergie aber nicht notwendigerweise durch präzise Synchronisation erfassen lassen. Das hier beschriebene Verfahren eignet sich daher insbesondere zu Berechnung eines robusten Maßes für Kommunikationsprozesse.

Das Ziel des erfindungsgemäßen Verfahrens ist es, ein Maß für einen Zusammenhang zwischen zwei gemessenen Aktivitäten anzugeben. Das Verfahren der imaginären Kohärenz verwendet als Bezugsgröße für dieses Maß die ursprünglich gemessenen Signale, während das erfindungsgemäße Verfahren als Bezugsgröße das erste frequenzbeschränkte Signal und den Anteil des zweiten frequenzbeschränkten Signals verwendet. Mit anderen Worten verwendet das erfindungsgemäße Verfahren als Bezugsgröße zwei Signale, von denen das zweite bereits um den Anteil bereinigt ist, der auch mit dem ersten Signal gemessen wird. Das Verfahren der imaginären Kohärenz unterschätzt aufgrund der gewählten Bezugsgröße im Vergleich zu dem erfindungsgemäßen Verfahren den tatsächlich bestehenden Zusammenhang zwischen zwei Aktivitäten.

Der zeitliche Verlauf der mit dem erfindungsgemäßen Verfahren bestimmten Maße für einen Zusammenhang der mit dem ersten und dem zweiten Sensor gemessenen Aktivitäten kann beispielsweise mit bekannten zeitlichen Verläufen von Maßen für den Zusammenhang zweier gemessener Aktivitäten verglichen werden, um einen Zustand des Gehirns beziehungsweise eine Zustand der Kommunikation des Gehirn zu bestimmen.

In einer bevorzugten Ausführungsform umfasst das Bestimmen des ersten frequenzbeschränkten Signals eine Fouriertransformation des ersten Signals in einer Vielzahl verschiedener Zeitfenster zur Bestimmung mindestens eines ersten komplexwertigen Fourierkoeffizienten für den jeweiligen Frequenzbereich in jedem Zeitfenster. Jeder Fourierkoeffizient enthält sowohl Amplituden- als auch Phaseninformationen über das erste Signal. Der mindestens eine komplexwertige Fourierkoeffizient bildet dann das erste frequenzbeschränkte Signal im jeweiligen Zeitfenster, und das erste frequenzbeschränkte Signal wird durch die mindestens einen komplexwertigen Fourierkoeffizienten in der Vielzahl von Zeitfenstern gebildet. Zeitlich benachbarte Zeitfenster können dabei beispielsweise zeitlich beabstandet, direkt aufeinanderfolgend oder auch überlappend ausgewählt werden. Im letzten Fall ist es besonders vorteilhaft, wenn es sich um ein gleitendes Zeitfenster handelt. Die Breite des Zeitfensters wird bevorzugt jeweils proportional zum Inversen der gewünschten Frequenzauflösung gewählt. Entsprechend kann auch verfahren werden, um das zweite frequenzbeschränkte Signal aus dem zweiten Signal zu bestimmen. Diese Ausführungsform ist besonders vorteilhaft, da die Berechnung des Anteils des zweiten frequenzbeschränkten Signals, der eine geringere lineare Korrelation mit dem ersten Signal aufweist als das zweite Signal, vereinfacht wird.

Ist das erste oder zweite Signal vor der Fouriertransformation noch nicht auf den ausgewählten Frequenzbereich eingeschränkt worden, so können nach der Fouriertransformation beispielsweise einfach die Fourierkoeffizienten ausgewählt werden, deren Frequenzen innerhalb des ausgewählten Frequenzbereiches liegen.

In jedem Fall können bei ausreichend hoher Frequenzauflösung nach der Fouriertransformation innerhalb des ausgewählten Frequenzbereichs in jedem Zeitfenster Fourierkoeffizienten für mehrere Frequenzen vorliegen. Die Fourierkoeffizienten des ersten frequenzbeschränkten Signals und des zweiten frequenzbeschränkten Signals liegen - wie auch im Falle nur eines Fourierkoeffizienten pro Frequenzbereich und Zeitfenster - jeweils paarweise für die gleichen Frequenzen vor. Mit anderen Worten, weist das erste Frequenzbeschränkte Signal Fourierkoeffizienten zu einer ausgewählten Frequenz auf, so weist auch das zweite frequenzbeschränkte Signal Fourierkoeffizienten zu dieser Frequenz auf. Die folgenden Schritte des oben beschriebenen Verfahrens können jetzt auf verschiedene Arten durchgeführt werden, von denen einige bevorzugte im Folgenden beispielhaft kurz erläutert werden.

Im Fall mehrerer Fourierkoeffizienten pro Zeitfenster kann beispielsweise in jedem Zeitfenster der Mittelwert der Fourierkoeffizienten für den jeweiligen Frequenzbereich gebildet werden und in dem Zeitfenster als erstes bzw. zweites frequenzbeschränktes Signal verwendet werden. Die nachfolgenden Schritte können dann wie oben beschrieben durchgeführt werden. Dieses Vorgehen ist vorteilhaft, wenn der Zusammenhang aller Aktivitäten in dem Frequenzbereich untersucht werden soll.

Alternativ können beispielsweise für jede Frequenz, für die Fourierkoeffizienten bestimmt worden sind, die Fourierkoeffizienten des ersten Signals zu einem ersten frequenzspezifischen Signal und die Fourierkoeffizienten des zweiten Signals zu einem zweiten frequenzspezifischen Signal zusammengefasst werden. Mit anderen Worten besteht in diesem Fall jedes frequenzbeschränkte Signal aus einer Vielzahl von frequenzspezifischen Signalen, von denen jedes aus denen zu der spezifischen Frequenz zugehörigen Fourierkoeffizienten in der Vielzahl von Zeitfenstern gebildet wird.

Auf dieser Basis kann die erste Einhüllende beispielsweise bestimmt werden, indem für jedes der frequenzspezifischen Signale eine eigene frequenzspezifische Einhüllende berechnet wird, wobei dann z.B. die verschiedenen frequenzspezifischen Einhüllenden zusammen die erste Einhüllende bilden oder aber deren Mittelwerte in jedem Zeitfenster die erste Einhüllende bilden. Eine Mittelung der Einhüllenden ist besonders vorteilhaft, wenn auch der Zusammenhang solcher Aktivitäten untersucht werden soll, die in überlappenden oder benachbarten Frequenzbändern ablaufen. Wird im Folgenden mit den frequenzspezifischen Einhüllenden weitergearbeitet, so können Zusammenhänge zwischen Aktivitäten, die verschiedenen frequenzspezifischen Signalen zugeordnet worden sind, nicht mit erfasst werden. Umgekehrt ist es vorteilhaft im Folgenden frequenzspezifische Einhüllende zu verwenden, wenn eine genaue Bestimmung des Zusammenhangs von Aktivitäten bei ausgewählten Frequenzen gewünscht ist.

Ebenso kann auch zum Bilden der zweiten Einhüllenden verfahren werden, wobei die Vorgehensweise für die erste Einhüllende und die zweite Einhüllende jeweils in Bezug auf die oben genannten Möglichkeiten bevorzugt identisch ist. Es wird folglich beispielsweise für jede Frequenz, für die ein erstes und ein zweites frequenzspezifisches Signal vorliegt, eine frequenzspezifische Einhüllende eines Anteil des zweiten frequenzspezifischen Signals berechnet, der eine geringere lineare Korrelation mit dem ersten für die gleiche Frequenz spezifischen Signal aufweist als das zweite frequenzspezifische Signal. Entsprechend dem Vorgehen zum Bestimmen der ersten Einhüllenden kann beispielsweise die Menge der zweiten frequenzspezifischen Einhüllenden die zweite Einhüllende bilden oder aber deren Mittelwert in jedem Zeitfenster.

Liegt im verbleibenden Schritt eine erste und eine zweite Einhüllende vor, die für jedes Zeitfenster jeweils nur einen Wert aufweisen, so kann das Maß für den Zusammenhang der Aktivitäten in der oben beschrieben Weise direkt zwischen diesen beiden Einhüllenden bestimmt werden. Bestehen die Einhüllenden jedoch jeweils aus einer Menge von mehreren frequenzspezifischen Einhüllenden, so kann beispielsweise für jede Frequenz, für die eine erste und eine zweite frequenzspezifische Einhüllende bestimmt worden sind, ein frequenzspezifisches Maß für den Zusammenhang der Aktivitäten nach den oben genannten Methoden berechnet werden. Als Maß für den Zusammenhang der Aktivitäten in dem Frequenzbereich kann beispielsweise die Menge der Maße verwendet werden oder aber deren Mittelwert.

In einer bevorzugten Ausführungsform entspricht der Anteil des zweiten frequenzbeschränkten Signals der Differenz zwischen dem zweiten frequenzbeschränkten Signal und dem mit einem reellwertigen Faktor multiplizierten ersten frequenzbeschränkten Signal, wobei der beispielsweise mittels der Methode der kleinsten Quadrate bestimmte reellwertige Faktor einen linearen Zusammenhang zwischen dem ersten frequenzbeschränkten Signal und dem zweiten frequenzbeschränkten Signal beschreibt. Mit anderen Worten ergibt sich der Anteil $y'$ des zweiten frequenzbeschränkten Signals $y$ aus $y' = y - f * x$, wobei $f$ den Faktor darstellt und $x$ das erste frequenzbeschränkte Signal ist. Im Fall von mehreren frequenzspezifischen Signalen werden die vorstehenden Schritte bevorzugt für jedes frequenzspezifische Signal separat durchgeführt.

Zur Bestimmung des Faktors $f$ aus den gemessenen Daten wird bevorzugt ein Zeitbereich festgelegt, für den davon ausgegangen werden kann, dass die an dem ersten Sensor und die an dem zweiten Sensor gemessenen Aktivitäten in einem festen Zusammenhang stehen. Der Zeitbereich muss wenigstens zwei Zeitpunkte umfassen, für die Messwerte aufgezeichnet worden sind. Der Zeitbereich kann beispielsweise dem für eine Fouriertransformation verwendeten Zeitfenster um jeden Messwert herum entsprechen oder auch dem gesamten Zeitraum, in dem das erste und das zweite Signal aufgezeichnet worden sind. Für jeden Messwert in dem festgelegten Zeitbereich wird angenommen, dass ein linearer Zusammenhang zwischen dem ersten und dem zweiten frequenzbeschränkten Signal aufgrund der gleichzeitigen Messung einer oder mehrerer von den gleichen Quellen erzeugten Teilaktivitäten an beiden Sensoren besteht.

Beispielsweise könnte eine untersuchte Person über einen Zeitbereich $T$ eine bestimmte Handlung ausrühren. Während die Handlung ausgeführt wird, werden einige Teilaktivitäten, die kontinuierlich auftreten, mit einer ersten variablen Intensität am ersten Sensor gemessen und mit einer zweiten variablen Intensität am zweiten Sensor gemessen. Das Verhältnis aus der ersten und der zweiten Intensität ist jedoch über den gesamten Zeitbereich $T$ konstant. Es besteht somit eine lineare Korrelation zwischen dem ersten frequenzbeschränkten Signal und dem zweiten frequenzbeschränkten Signal zumindest in dem Umfang, in dem sie von der oder den gleichen Teilaktivitäten erzeugt werden. Bestimmt man nun über den gesamten Zeitbereich T eine lineare Korrelation zwischen dem ersten und dem zweiten frequenzbeschränkten Signal, so gibt diese den Anteil des ersten frequenzbeschränkten Signals an, der kontinuierlich zu dem zweiten frequenzbeschränkten Signal beiträgt. Da der relative Anteil über den gesamten Zeitbereich $T$ konstant ist, muss für alle in den Zeitbereich $T$ fallenden Signalwerte nur ein einziger reellwertiger Faktor $f$ bestimmt werden. Der Faktor $f$ kann beispielsweise mittels der Methode der kleinsten Quadrate berechnet werden und ergibt sich aus den Werten $x_t$ des ersten frequenzbeschränkten Signals und den Werten $y_t$ des zweiten frequenzbeschränkten Signals zu den Zeitpunkten $t$ in dem Zeitbereich $T$ durch

$$f = \mathrm{real}\left(\sum_{t\in T} x_t\,\underline{y}_t \,\Big/\, \sum_{t\in T} x_t\,\underline{x}_t\right), \qquad\qquad \text{(Gleichung 1)}$$

wobei der Unterstrich das komplex konjugierte und *real* den Realteil einer komplexen Zahl bezeichnet.

Dieses Verfahren ist besonders vorteilhaft, da der Zeitbereich, über den eine lineare Korrelation zwischen dem ersten und dem zweiten frequenzbeschränkten Signal aufgrund der Messung der gleichen Teilaktivitäten an beiden Sensoren besteht, in Abhängigkeit von der Untersuchung gewählt werden kann. Sollen beispielsweise die Zusammenhänge zwischen den an den beiden Sensoren gemessenen Aktivitäten für eine kurze Handlung bestimmt werden, so wird der Faktor *f* nur für den Zeitbereich *T* bestimmt, in dem die Handlung abläuft, da nur in diesem Zeitraum davon ausgegangen werden kann, dass die gleichen Teilaktivitäten kontinuierlich an beiden Sensoren gemessen werden. Werden andererseits beispielsweise die Zusammenhänge bei einer schlafenden Person bestimmt, so sind die an beiden Sensoren gleichzeitig gemessenen Teilaktivitäten über einen deutlich längeren Zeitbereich regelmäßig aktiv. Entsprechend länger kann auch der Zeitbereich *T* gewählt werden, in dem der Faktor *f* bestimmt wird. Es ist auch vorstellbar, den Zeitbereich flexibel anzupassen. So können beispielsweise dann, wenn die Signale mittels MEG aufgenommen werden, die Zeitbereiche um einen Messwert herum jeweils so lang gewählt werden, wie sich die Position der Sensoren relativ zum Kopf bzw. der die Aktivitäten erzeugenden Quellen nicht verändert hat. Wenn sich die relative Position der Sensoren zum Kopf bzw. zu den die Aktivitäten erzeugenden Quellen verändert, dann verändert sich auch der Abstand und/oder die Orientierung der Sensoren zu den die Aktivitäten erzeugenden Quellen und damit der Anteil zu dem die von zwei beabstandet angeordneten Sensoren gemessenen Aktivitäten von den gleichen Quellen erzeugt werden.

In einer alternativen bevorzugten Ausführungsform entspricht der Anteil des zweiten frequenzbeschränkten Signals dem Anteil des zweiten frequenzbeschränkten Signals, dessen Phase von der Phase des ersten frequenzbeschränkten Signals abweicht und insbesondere um einen bestimmten Wert oder um Werte aus einem bestimmten Winkelbereich abweicht. Es ist besonders bevorzugt, wenn der Anteil des zweiten frequenzbeschränkten Signals bestimmt wird, dessen Phase um 90° von der Phase des ersten frequenzbeschränkten Signals abweicht. Diese Vorgehensweise ist besonders vorteilhaft, da die Berechnung des Anteils des zweiten frequenzbeschränkten Signals auf den jeweiligen Messzeitpunkt beschränkt ist. Daher werden nur sehr geringe Anforderungen an die Stationarität gestellt. Es werden nur die Messwerte innerhalb eines Zeitfenster benötigt, das notwendig ist, um die Phase des ersten und des zweiten frequenzbeschränkten Signals zu bestimmen, und das beispielsweise eines der oben genannten Zeitfenster sein kann. Weiterhin ist diese Ausführungsform besonders für Messdaten geeignet, bei denen nicht sichergestellt werden kann, dass sich die relative Position der Sensoren zum Gehirn nicht verändert, wie dies zum Beispiel in der MEG der Fall ist. Wird der Anteil des zweiten Signals berechnet, der eine um 90° von der Phase des ersten Signals abweichende Phase aufweist, so wird dieser Anteil nur von solchen Aktivitäten erzeugt, die nicht auch am ersten Sensor gemessen wurden.

Umfasst der Schritt des Bestimmens der frequenzbeschränkten Signale eine Fouriertransformation des ersten und des zweiten Signals, so kann in einer weiteren alternativen Ausführungsform für jedes Zeitfenster der Anteil des zweiten frequenzbeschränkten Signals als Projektion des zweiten frequenzbeschränkten Signals auf die zum ersten frequenzbeschränkten Signal orthogonale Richtung in der komplexen Ebene bestimmt werden. Hierdurch wird mit den obigen Vorteilen nur der um 90° phasenverschobene Signalanteil berücksichtigt. Mit anderen Worten erfolgt für jedes Zeitfenster eine Berechnung eines Anteils des zweiten frequenzbeschränkten Signals, der orthogonal zu dem ersten frequenzbeschränkten Signal steht. Soll beispielsweise der Anteil Y' eines frequenzbeschränkten Signals Y, das aus den Fourierkoeffizienten des Signals y besteht, bestimmt werden, der eine von dem frequenzbeschränkten Signal X, das entsprechend aus den Fourierkoeffizienten des Signals x besteht, um 90° verschiedene Phase aufweist, so kann dies beispielsweise geschehen indem Y' = *imag*(Y * $\underline{X}$/|X|) berechnet wird, wobei *imag* den imaginären Teil und die beiden senkrechten Striche den Betrag einer komplexen Zahl bezeichnen. Liegen in jedem Zeitfenster Fourierkoeffizienten bei unterschiedlichen Frequenzen vor, so wird für jede dieser Frequenzen der Anteil des jeweiligen Fourierkoeffizienten des zweiten Signals bestimmt, der orthogonal zu dem Fourierkoeffizienten bei der gleiche Frequenz des ersten Signals ist. Diese bevorzugte Ausführungsform ist besonders einfach und schnell ausführbar, da nach der Fouriertransformation nur elementare Rechenoperationen auszuführen sind. Weiterhin weist auch diese Ausführungsform die bereits genannten Vorteile eines besonders kurzen Vergleichzeitraums auf.

In einer bevorzugten Ausführungsform wird ferner eine dritte Einhüllenden des zweiten frequenzbeschränkten Signals und eine vierte Einhüllende eines Anteils des ersten frequenzbeschränkten Signals bestimmt, der eine geringere lineare Korrelation mit dem zweiten frequenzbeschränkten Signal aufweist als das erste frequenzbeschränkte Signal. Mit anderen Worten sind in Bezug auf die dritte und die vierte Einhüllende die Rolle des ersten und des zweiten Signals im Vergleich zu der ersten und zweiten Einhüllenden vertauscht. Es wird dann ein Maß für den Zusammenhang, wie z.B. ein Maß für eine Korrelation, zwischen der dritten Einhüllenden und der vierten Einhüllenden als ein weiteres Maß für einen Zusammenhang bzw. eine Korrelation zwischen den an dem ersten und dem zweiten Sensor gemessenen Aktivitäten bestimmt, und schließlich werden das aus der ersten Einhüllenden und der zweiten Einhüllenden ermittelte Maß für den Zusammenhang und das aus der dritten Einhüllenden und der vierten Einhüllenden ermittelte Maß für den Zusammenhang gemittelt. Das gemittelte Maß für den Zusammenhang ist symmetrisch und ggf. robuster als die einzelnen Maße.

Die Bestimmung der vierten Einhüllenden bzw. die Bestimmung des Anteils des ersten frequenzbeschränkten Signals, der eine geringere lineare Korrelation mit dem zweiten frequenzbeschränkten Signal aufweist als das erste frequenzbeschränkte Signal, kann in derselben Weise erfolgen, wie es oben für die zweite Einhüllende bzw. die Bestimmung des Anteils des zweiten frequenzbeschränkten Signals, der eine geringere lineare Korrelation mit dem ersten frequenzbeschränkten Signal aufweist als das zweite frequenzbeschränkte Signal, wobei das erste und das zweite frequenzbeschränkte Signal die Rollen tauschen.

Die Aufgabe wird weiterhin von einem Verfahren zur Bestimmung eines Maßes für einen Zusammenhang zwischen den von mindestens drei Sensoren gleichzeitig gemessenen Aktivitäten gelöst. Dabei wird mittels des oben detailliert erläuterten Verfahrens für jedes Paar der Sensoren ein paarspezifisches Maß für einen Zusammenhang zwischen den gemessenen Aktivitäten bestimmt. Anschließend werden die so gewonnenen paarspezifischen Zusammenhangsmaße in einer Matrix angeordnet, die das Maß für einen Zusammenhang zwischen den gemessenen Aktivitäten darstellt. Dieses erfindungsgemäße Verfahren ist besonders vorteilhaft, da aus der Analyse der Einträge in der Matrix, die den Vergleich der Signale vieler Sensoren umfasst, robustere und auch weiter reichende Aussagen über den Zusammenhang verschiedener Aktivitäten des Gehirns gemacht werden können.

Das beschriebene Verfahren und seine vorteilhaften Ausgestaltungen können bevorzugt mit Hilfe einer Vorrichtung bzw. eines Systems mit wenigstens einem ersten elektrischen oder magnetischen Sensor und einem zweiten elektrischen oder magnetischen Sensor und mit einer Datenverarbeitungsanlage durchgeführt werden, wobei die Datenverarbeitungsanlage angepasst ist, um die oben beschriebenen Verfahrensschritte durchzuführen. Insbesondere weist die Vorrichtung eine Verbindung zum Übertragen der von den Sensoren ausgegebenen Signale an die Datenverarbeitungseinheit auf. Es ist möglich, dass sowohl die Sensorelektronik als auch die Datenverarbeitungsanlage die Signale bereits einer Vorverarbeitung unterziehen, bevor sie nach dem oben stehenden Verfahren verarbeitet werden. Es ist weiterhin möglich, dass die Vorrichtung eine Vielzahl von Sensoren aufweist.

Die vorliegende Erfindung wird im Folgenden anhand von ein Ausführungsbeispiel darstellenden Zeichnungen erläutert. Die Zeichnungen zeigen in

Fig. 1     eine schematische Darstellung einiger Schritte des erfindungsgemäßen Verfahrens umfassend die Messung zweier Aktivitäten mit zwei Sensoren, die Bestimmung zweier frequenzbeschränkter Signale und die Bestimmung einer ersten, einer zweiten, einer dritten und einer vierten Einhüllenden und in

Fig. 2     eine schematische Darstellung einer Bestimmung eines Anteils des zweiten Signals.

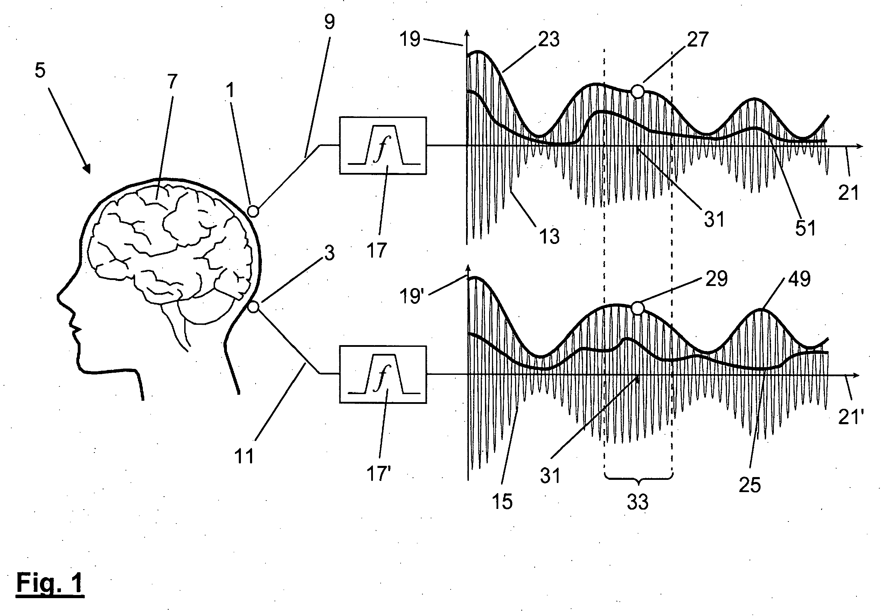

In Figur 1 ist eine schematische Elektroenzephalografie- (EEG) Messung dargestellt, bei der ein erster Sensor 1 und ein zweiter Sensor 3 auf der Oberfläche eines Kopfes 5 voneinander beabstandet angeordnet sind. Der erste Sensor 1 misst eine erste Aktivität des Gehirns 7 und gibt ein erstes zeitabhängiges Signal 9 aus. Entsprechend misst der zweite Sensor 3 eine Aktivität des Gehirns 7 und gibt ein zweites zeitabhängiges Signal 11 aus.

Die vollständige EEG-Messung, die dem hier dargestellten Ausführungsbeispiel zugrunde liegt, verwendet eine Vielzahl von Einzelsensoren. Diese Einzelsensoren sind alle auf der Oberfläche des Kopfes 5 voneinander beabstandet angeordnet. Jeder der Einzelsensoren misst eine Aktivität des Gehirns und gibt ein zeitabhängiges Signal aus. In einem ersten Vorverarbeitungsschritt werden die von den Einzelsensoren ausgegebenen zeitabhängigen Signale von Artefakten bereinigt und eine Quellenlokalisation durchgeführt. Bei der Quellenlokalisation wird aus den relativen Signalintensitäten der an den jeweiligen Einzelsensoren gemessenen Aktivitäten eine räumliche Verteilung von Quellen im Gehirn 7 geschätzt, die die gemessenen elektrischen Aktivitäten erzeugen könnten. Jeder dieser Quellen wird nachfolgend ein zeitabhängiges Signal zugeordnet, das sich aus einer Linearkombination der ausgegebenen und von Artefakten bereinigten Signale der Einzelsensoren ergibt.

In diesem Ausführungsbeispiel entsprechen die in Figur 1 schematisch dargestellten zeitabhängigen Signale 9, 11 jeweils einem zeitabhängigen Signal einer dieser Quellen, das heißt, die von den Sensoren 1, 3 ausgegeben Signale 9, 11 sind eine Linearkombination der von den Einzelsensoren gemessenen Signale. Es ist jedoch auch denkbar, dass die nachfolgenden Schritte mit den von den Einzelsensoren ausgegebenen und ggf. von Artefakten bereinigten zeitabhängigen Signalen direkt durchgeführt werden.

Im nachfolgenden Schritt werden zwei Frequenzebereiche des ersten Signals 1 und des zweiten Signals 3 ausgewählt. Bei dem ersten Frequenzbereich handelt es sich um den sogenannten "Theta"-Frequenzbereich, der von 4 Hz bis 8 Hz reicht. Der zweite Frequenzbereich umfasst den "Alpha"- und den "Beta"-Frequenzbereich und reicht damit von 8 Hz bis 30 Hz. Es ist allerdings auch denkbar, dass nur ein Frequenzbereich ausgewählt wird oder dass mehr als zwei Frequenzbereiche ausgewählt werden können. So ist es beispielsweise vorstellbar, den "Alpha"-Frequenzbereich (8 bis 13 Hz) und den "Beta"-Frequenzbereich (13 bis 30 Hz) getrennt zu untersuchen. Die nachfolgend beschriebenen Schritte werden in jedem Fall für jeden der ausgewählten Frequenzbereiche einzeln durchgeführt.

Zunächst wird aus dem ersten Signal 9 ein erstes frequenzbeschränktes Signal 13 und aus dem zweiten Signal 11 ein zweites frequenzbeschränktes Signal 15 bestimmt. Dies kann beispielsweise geschehen, in dem ein Bandpassfilter 17, 17' auf das erste Signal 9 und das zweite Signal 11 angewendet wird. In Figur 1 ist für einen zeitlich begrenzten Abschnitt die zeitliche Veränderung der Signalstärke 19, 19' des ersten und des zweiten frequenzbeschränkten Signals 13, 15 jeweils entlang einer Zeitachse 21, 21' aufgetragen.

Von dem ersten frequenzbeschränkten Signal 13 wird eine erste Einhüllende 23 bestimmt, indem zu jedem Zeitpunkt t der Logarithmus aus dem Quadrat des Absolutbetrags des jeweiligen Wertes $x_t$ des ersten frequenzbeschränkten Signals 23 berechnet wird. Mit anderen Wort ergibt sich die Einhüllende 23, indem $log(|x_t|^2)$ berechnet wird, wobei $log$ den Logarithmus bezeichnet und die senkrechten Striche den Absolutbetrag. Die in Figur 1 dargestellte Einhüllende 23 zeigt lediglich eine schematische Darstellung der Einhüllenden 23 des ersten frequenzbeschränkten Signals 13, die verzerrt wurde, damit sie auch in der graphischen Darstellung eine Einhüllende 23 des ersten frequenzbeschränkten Signals 13 bildet. Im Allgemeinen beeinflusst eine Umskalierung der Einhüllenden 23 mit einem konstanten Faktor den Wert des zu bestimmenden Maßes für einen Zusammenhang nicht.

Des Weiteren wird eine Einhüllende 25 eines Anteils des zweiten frequenzbeschränkten Signals 15 bestimmt, der eine geringere lineare Korrelation mit dem ersten frequenzbeschränkten Signal 13 aufweist als das zweite frequenzbeschränkte Signal 15. In den nachfolgend beschriebenen Schritten wird hierzu zunächst eine Fouriertransformation des ersten und des zweiten frequenzbeschränkten Signals 13, 15 durchgeführt. Es ist jedoch auch möglich, die Einhüllende 25 des Anteils direkt aus den zeitabhängigen Signalen 13, 15 zu bestimmen.

Um die Fouriertransformation durchzuführen, wird zunächst für jeden Wert 27, 29 des ersten bzw. zweiten frequenzbeschränkten Signals 13, 15, die zu einem Zeitpunkt 31 aufgezeichnet wurden, ein Zeitfenster 33 festgelegt. Die zeitliche Breite des Zeitfensters 33 ist dabei umgekehrt proportional zur gewünschten Frequenzauflösung der Fouriertransformation. In der Regel wird der zeitliche Abstand zwischen zwei benachbarten Werten 27, 29 des ersten bzw. des zweiten frequenzbeschränkten Signals 13, 15 kleiner sein, als die gewählte Breite des Zeitfensters 33. Um für jeden der Werte 27, 29 eine Fouriertransformation durchführen zu können, muss folglich eine Vielzahl von sich überlappenden Zeitfenstern 33 ausgewählt werden. Sind die Zeitfenster 33 alle gleich breit, so wird die Fouriertransformation in einem gleitenden Zeitfenster 33 durchgeführt.

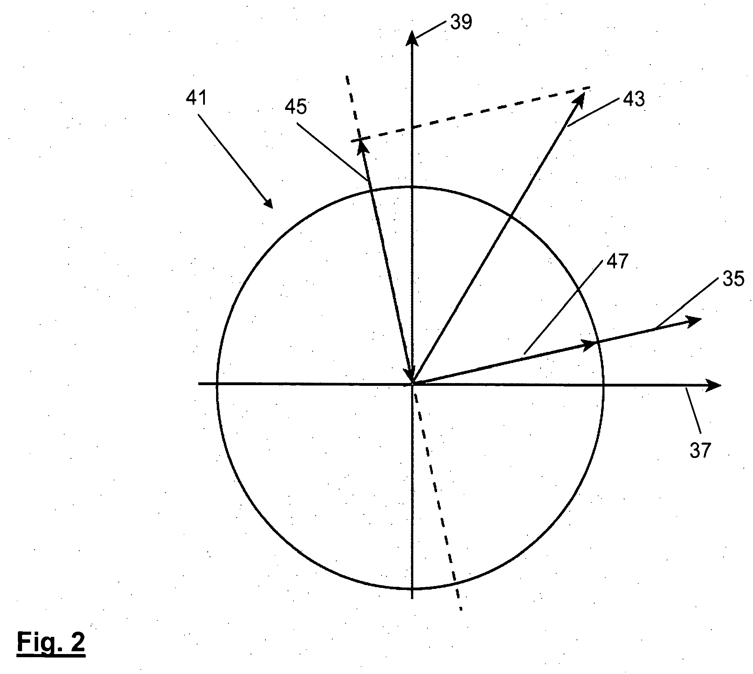

In dem Zeitfenster 33 wird eine Fouriertransformation des ersten frequenzbeschränkten Signals 13 und des zweiten frequenzbeschränkten Signals 15 durchgeführt. Aus der Fouriertransformation des ersten frequenzbeschränkten Signals 13 erhält man einen ersten komplexwertigen Fourierkoeffizienten 35, der in Figur 2 in der von einer reellen Achse 37 und einer imaginären Achse 39 aufgespannten komplexen Ebene 41 dargestellt ist. Entsprechend erhält man aus der Fouriertransformation des zweiten frequenzbeschränkten Signals 15 einen zweiten komplexwertigen Fourierkoeffizienten 43. Die Länge der Vektoren, die die Fourierkoeffizienten 35, 43 in Figur 2 darstellen, entspricht dabei dem Absolutbetrag der Fourierkoeffizienten 35, 43 und der Winkel, der von dem jeweiligen Vektor und der reellen Achse 37 eingeschlossen wird, der Phase der Fourierkoeffizienten 41, 43.

Um den Anteil des zweiten frequenzbeschränkten Signals 15 zu bestimmen, der eine geringere lineare Korrelation mit dem ersten frequenzbeschränkten Signal 13 aufweist als das zweite frequenzbeschränkte Signal 15, kann auf verschiedene Weise vorgegangen werden. In Figur 2 ist ein Anteil 45 des zweiten Fourierkoeffizienten 43 dargestellt, der eine Phasendifferenz von 90° zu dem ersten Fourierkoeffizienten 35 aufweist.

Um den Anteil 45 zu bestimmen kann beispielsweise wie folgt vorgegangen werden. Zunächst wird der Einheitsvektor 47, dass heißt, ein Vektor mit der Länge eins, bestimmt, der in die gleiche Richtung in der komplexen Ebene weist wie der erste Fourierkoeffizient 35. Bildet man das Produkt des komplex konjugierten des Einheitsvektors 47 mit dem zweiten Fourierkoeffizienten 43 und betrachtet lediglich den Imaginärteil des Produktes, so erhält man den Anteil 45. Anders ausgedrückt ergibt sich das den Anteil 45 bezeichnende $Y'$ in dem man $Y' = imag( Y \cdot \underline{X} / |X|)$ berechnet, wobei $X$ für den ersten Fourierkoeffizienten 35 steht, $Y$ für den zweiten Fourierkoeffizienten 43, $imag$ für den Imaginärteil einer komplexen Zahl und ein Unterstrich für einen komplex konjugierten Wert. Den Einheitsvektor 47 zu dem ersten Fourierkoeffizienten 35 erhält man, in dem man $X / |X|$ bestimmt.

In einer alternativen Ausführungsform wird der Anteil 45 bestimmt, indem man von dem zweiten Fourierkoeffizienten 43 den mit einem reellwertigen Faktor $f$ multiplizierten ersten Fourierkoeffizienten 35 abzieht. Mit anderen Worten berechnet man $Y'$ durch $Y' = Y - f * X$. Der Faktor f kann beispielsweise bestimmt werden, in dem man das Produkt aus dem ersten Fourierkoeffizienten 35 und dem komplex konjugierten des zweiten Fourierkoeffizienten 43 durch das Betragsquadrat des ersten Fourierkoeffizienten 35 teilt und von dem Produkt den Realteil bestimmt. Anders dargestellt ist $f = real (X * \underline{Y} / (X * \underline{X}))$, wobei $real$ den Realteil einer komplexen Zahl bezeichnet.

In einer weiteren alternativen Ausführungsform wird der Faktor $f$ für einen Zeitbereich $T$ bestimmt, der vorher festzulegen ist. Für jeden Zeitpunkt 31 innerhalb des Zeitbereichs $T$ für den Fourierkoeffizienten 35, 43 vorliegen, wird das Produkt aus dem ersten Fourierkoeffizienten 35 und dem komplex konjugierten des zweiten Fourierkoeffizienten gebildet. Der Realteil der Summe aller Produkte dividiert durch die Summe der Betragsquadrate der ersten Fourierkoeffizienten 35 zu den entsprechend Zeitpunkten 31 ergibt den Faktor *f*. Anders dargestellt ist

$$f = \mathrm{real}\left(\sum_{t \in T} x_t \underline{y}_t \bigg/ \sum_{t \in T} x_t \underline{x}_t\right),$$

(Gleichung 2)

wobei t einen Zeitpunkt 31 bezeichnet. Die vorstehende Ausführungsform ist besonders vorteilhaft, da sie erlaubt, den Zusammenhang zwischen einer mit einem ersten Sensor 1 gemessenen Aktivität und einer mit einem zweiten Sensor 3 gemessenen Aktivität über einen längeren Zeitbereich zu bestimmen. Dies ist insbesondere daher vorteilhaft, da die Zusammenhänge nicht von kurzfristigen Veränderungen in den frequenzbeschränkten Signalen 13, 15 überlagert werden. Es ergibt sich somit ein robusteres Maß für den Zusammenhang als bei der imaginären Kohärenz.

Zur Bestimmung der zweiten Einhüllenden 25 des Anteils der zweiten frequenzbeschränkten Signals 15 wird der Anteil 45 der zweiten Fourierkoeffizienten 43 in einer Vielzahl von Zeitfenstern 33 beispielsweise nach einer der oben beschriebenen Methoden berechnet. Die zweite Einhüllende 25 ergibt sich dann, in dem man die Einhüllende 25 der Anteile 45 berechnet. Dies kann nach der gleichen Methode erfolgen, mit der bereits die erste Einhüllende 23 des ersten frequenzbeschränkten Signals 13 berechnet wurde.

Um einen Zusammenhang zwischen der mit dem ersten Sensor 1 gemessenen Aktivität und der mit dem zweiten Sensor 3 gemessenen Aktivität zu bestimmen, wird in einem weiteren Schritt eine lineare Korrelation zwischen der ersten Einhüllenden 23 und der zweiten Einhüllenden 25 berechnet. Es ist auch denkbar, dass ein anderes Maß zur Bestimmung des Zusammenhangs verwendet wird.

In weiteren Schritten, die hier nicht im Detail dargestellt werden, wird in dem erfindungsgemäßen Ausführungsbeispiel zudem eine Korrelation einer dritten Einhüllenden 49 des zweiten frequenzbeschränkten Signals 15 und einer vierten Einhüllenden 51 eines Anteils des ersten frequenzbeschränkten Signals 13 berechnet, der eine geringere lineare Korrelation mit dem zweiten frequenzbeschränkten Signal 15 aufweist als das erste frequenzbeschränkte Signal 13. Hierzu wird nach den oben beschriebenen Schritten vorgegangen, wobei die Rolle des ersten und des zweiten frequenzbeschränkten Signals 13, 15 vertauscht sind. Auch die auf diese Weise bestimmte lineare Korrelation stellt ein Maß für einen Zusammenhang zwischen der an dem ersten Sensor 1 gemessenen Aktivität und der an dem zweiten Sensor 3 gemessenen Aktivität dar.

Zuletzt wird zu jedem Zeitpunkt 31 der Mittelwert aus der zwischen der ersten und der zweiten Einhüllenden 23, 25 berechneten Korrelation und der zwischen der dritten und der vierten Einhüllenden 49, 51 berechneten Korrelation gebildet. Dieser Mittelwert gibt ebenfalls Auskunft über den Zusammenhang zwischen der an dem ersten Sensor 1 gemessenen Aktivität und der an dem zweiten Sensor 3 gemessenen Aktivität. Dieses Maß für den Zusammenhang ist besonders vorteilhaft, da es sich um ein symmetrisches Maß handelt.

Das erfindungsgemäße Ausführungsbeispiel weist eine Reihe von Vorteilen gegenüber dem Stand der Technik auf, da es keine scheinbaren Zusammenhänge berücksichtigt, die lediglich auf einer Messung der gleichen Teilaktivitäten an beiden Sensoren 1 ,3 beruhen, und existierende Zusammenhänge nicht durch Veränderungen im Phasenunterschied zwischen dem ersten Signal 9 und dem zweiten Signal 11 verdeckt werden. Zudem stellt das beanspruchte Verfahren im Gegensatz zur imaginären Kohärenz ein robustes Maß dar, das nicht von schwer zu messenden und eventuell nicht vorhandenen präzisen Phasenrelationen abhängt, sondern von der leichter zu messenden Co-Variation der Signalenergie, die nach Ansicht des Anmelders eine viel allgemeiner auftretende und leichter zu erfassende Signatur von Kommunikationsprozessen ist.

Zudem weist das erfindungsgemäße Ausführungsbeispiel gegenüber den aus dem Stand der Technik bekannten funktionellen Bildgebungsverfahren eine deutliche höhere zeitliche Auflösung bei deutlichen geringerem apparativen und damit auch kostenmäßigen Aufwand auf. Da der Zusammenhang nicht zwischen den ausgegebenen Signalen selbst gebildet wird, sondern zwischen der Einhüllenden 23, 49 eines ersten Signals 13 und der Einhüllenden 25, 51 eines Anteils eines zweiten Signals 15, der ein geringere lineare Korrelation mit dem ersten Signal 13 aufweist als das zweite Signal 15, werden dabei die sonst regelmäßig von MEG und EEG Messungen festgestellten falschen Korrelationen vermieden, die lediglich darauf beruhen, dass die gleiche Teilaktivität an beiden Sensoren 1, 3 gemessen wurde.

**Patentansprüche**

1. Verfahren zur Bestimmung eines Maßes für einen Zusammenhang zwischen einer mit einem ersten Sensor (1) gemessenen elektrischen oder magnetischen Aktivität des Gehirns (7) und einer mit einem von dem ersten Sensor (1) beabstandet angeordneten zweiten Sensor (3) gemessenen elektrischen oder magnetischen Aktivität des Gehirns (7), wobei bei dem Verfahren

   ein von dem ersten Sensor (1) ausgegebenes erstes zeitabhängiges Signal (9) und ein von dem zweiten Sensor (3) ausgegebenes zweites zeitabhängiges Signal (11) zeitgleich aufgezeichnet werden,
ein oder mehrere Frequenzbereiche des ersten Signals (1) und des zweiten Signals (3) ausgewählt werden, die für die beiden Signale identisch sind und
in jedem ausgewählten Frequenzbereich ein Zusammenhang zwischen dem ersten Signal (1) und dem zweiten Signal (3) mit den folgenden Schritten bestimmt wird:

- Bestimmen eines auf den jeweiligen Frequenzbereich beschränkten ersten frequenzbeschränkten Signals (13) aus dem ersten Signal (9) und eines auf den jeweiligen Frequenzbereich beschränkten zweiten frequenzbeschränkten Signals (15) aus dem zweiten Signal (11),
- Bestimmen einer ersten Einhüllenden (23) des ersten frequenzbeschränkten Signals (13),
- Bestimmen einer zweiten Einhüllenden (25) eines Anteils des zweiten frequenzbeschränkten Signals (15), wobei der Anteil eine geringere lineare Korrelation mit dem ersten frequenzbeschränkten Signal (13) aufweist als das zweite frequenzbeschränkte Signal (15), und
- Bestimmen eines Maßes für den Zusammenhang zwischen der ersten Einhüllenden (23) und der zweiten Einhüllenden (25) als Maß für einen Zusammenhang zwischen der mit dem ersten Sensor (1) gemessenen elektrischen oder magnetischen Aktivität des Gehirns (7) und der mit dem zweiten Sensor (3) gemessenen elektrischen oder magnetischen Aktivität des Gehirns (7).

2. Verfahren nach Anspruch 1, bei dem das Bestimmen des ersten frequenzbeschränkten Signals (13) für jeden Frequenzbereich eine Fouriertransformation des ersten Signals (9) in einer Vielzahl verschiedener Zeitfenster (33) zur Bestimmung mindestens eines ersten komplexwertigen Fourierkoeffizienten (35) für den jeweiligen Frequenzbereich in jedem Zeitfenster (33) umfasst, wobei die entsprechenden komplexwertigen Fourierkoeffizienten (35) das erste frequenzbeschränkte Signal bilden (13), und
bei dem das Bestimmen des zweiten frequenzbeschränkten Signals (15) für jeden Frequenzbereich eine Fouriertransformation des zweiten Signals (11) in den Zeitfenstern (33) zur Bestimmung mindestens eines zweiten komplexwertigen Fourierkoeffizienten (43) für den jeweiligen Frequenzbereich in jedem Zeitfenster (33) umfasst, wobei die entsprechenden komplexwertigen Fourierkoeffizienten (43) das zweite frequenzbeschränkte Signal (15) bilden.

3. Verfahren nach Anspruch 1 oder 2, bei dem der Anteil des zweiten frequenzbeschränkten Signals (15) der Differenz zwischen dem zweiten frequenzbeschränkten Signal (15) und dem mit einem Faktor multiplizierte ersten frequenzbeschränkten Signal (13) entspricht, wobei der Faktor einen linearen Zusammenhang zwischen dem ersten frequenzbeschränkten Signal (13) und dem zweiten frequenzbeschränkten Signal (15) beschreibt.

4. Verfahren nach Anspruch 1 oder 2, bei dem der Anteil des zweiten frequenzbeschränkten Signals (15) dem Anteil des zweiten frequenzbeschränkten Signals (15) entspricht, dessen Phase von der Phase des ersten frequenzbeschränkten Signals (13) abweicht.

5. Verfahren nach Anspruch 4, bei dem der Anteil des zweiten frequenzbeschränkten Signals (15) bestimmt wird, dessen Phase um 90° von der Phase des ersten frequenzbeschränkten Signals (13) abweicht.

6. Verfahren nach Anspruch 2, bei dem für jedes Zeitfenster (33) der Anteil des zweiten frequenzbeschränkten Signals (15) als Projektion des entsprechenden zweiten frequenzbeschränkten Signals (15) auf die zum entsprechenden ersten frequenzbeschränkten Signal (13) orthogonale Richtung in der komplexen Ebene (41) bestimmt wird.

7. Verfahren nach einem der vorhergehenden Ansprüche, **dadurch gekennzeichnet, dass** das Bestimmen eines Zusammenhangs zwischen der mit einem ersten Sensor (1) und der mit einem zweiten Sensor (3) gemessenen Aktivität des Gehirns (7) in jedem ausgewählten Frequenzbereich weiterhin die folgenden Schritte aufweist:

- Bestimmen einer dritten Einhüllenden (49) des zweiten frequenzbeschränkten Signals (15),
- Bestimmen einer vierten Einhüllenden (51) eines Anteils des ersten frequenzbeschränkten Signals (13), wobei der Anteil eine geringere lineare Korrelation mit dem zweiten frequenzbeschränkten Signal (15) aufweist als das erste frequenzbeschränkte Signal (13),
- Bestimmen eines Maßes für den Zusammenhang zwischen der dritten Einhüllenden (49) und der vierten Einhüllenden (51) und
- Bestimmen eines mittleren Maßes für den Zusammenhang zwischen der mit dem ersten Sensor (1) gemessenen elektrischen oder magnetischen Aktivität des Gehirns (7) und der mit dem zweiten Sensor (3) gemessenen elektrischen oder magnetischen Aktivität des Gehirns (7) aus dem Maß für den Zusammenhang zwischen der ersten Einhüllenden (23) und der zweiten Einhüllenden (25) und aus dem Maß für den Zusammenhang zwischen der dritten Einhüllenden (49) und der vierten Einhüllenden (51).

8. Verfahren nach Anspruch 7, bei dem der Anteil des ersten frequenzbeschränkten Signals (13) der Differenz zwischen dem ersten frequenzbeschränkten Signal (13) und dem mit einem Faktor multiplizierte zweiten frequenzbeschränkten Signal (15) entspricht, wobei der Faktor einen linearen Zusammenhang zwischen dem zweiten frequenzbeschränkten Signal (15) und dem ersten frequenzbeschränkten Signal (13) beschreibt.

9. Verfahren nach Anspruch 7, bei dem der Anteil des ersten frequenzbeschränkten Signals (13) dem Anteil des ersten frequenzbeschränkten Signals (13) entspricht, dessen Phase von der Phase des zweiten frequenzbeschränkten Signals (15) abweicht, wobei die Abweichung des Anteils der Phase des ersten frequenzbeschränkten Signals (13) von der Phase des zweiten frequenzbeschränkten Signals (15) vorzugsweise 90° beträgt.

10. Verfahren nach Anspruch 7 sofern abhängig von Anspruch 2, bei dem für jedes Zeitfenster (33) der Anteil des ersten frequenzbeschränkten Signals (13) als Projektion des entsprechenden ersten frequenzbeschränkten Signals (13) auf die zum entsprechenden mindestens einen zweiten frequenzbeschränkten Signal (15) orthogonale Richtung in der komplexen Ebene (41) bestimmt wird.

11. Verfahren nach einem der vorhergehenden Ansprüche, bei dem am ersten Sensor (1) und am zweiten Sensor (3) die elektrische Aktivität des Gehirns (7) mittels Elektroenzephalografie (EEG) oder Elektrocorticogramm (ECoG), oder die magnetische Aktivität des Gehirns (7) mittels Magnetoenzephalografie (MEG) gemessen wird.

12. Verfahren nach einem der vorhergehenden Ansprüche, **dadurch gekennzeichnet, dass** der erste Sensor (1) eine Mehrzahl von Einzelsensoren aufweist, wobei das erste zeitabhängige Signal (9) aus einer Linearkombination der von den Einzelsensoren des ersten Sensors (1) ausgegebenen Signale gebildet wird, und
dass der zweite Sensor (3) eine Mehrzahl von Einzelsensoren aufweist, wobei das zweite zeitabhängige Signal (11) aus einer Linearkombination der von den Einzelsensoren des zweiten Sensors (3) ausgegebenen Signale gebildet wird.

13. Verfahren nach Anspruch 12, wobei aus den Signalen der Einzelsensoren des ersten Sensors (1) mittels Quellenlokalisation ein Signal einer ersten Quelle bestimmt wird, das als erstes zeitabhängiges Signal (9) des ersten Sensors (1) ausgegeben wird, und
wobei aus den Signalen der Einzelsensoren des zweiten Sensors (3) mittels Quellenlokalisation ein Signal einer zweiten Quelle bestimmt wird, das als zweites zeitabhängiges Signal (11) des zweiten Sensors (3) ausgegeben wird.

14. Verfahren zur Bestimmung eines Maßes für einen Zusammenhang zwischen einer Vielzahl von elektrischen oder magnetischen Aktivitäten des Gehirns (7), bei dem mit Hilfe einer Vielzahl von Sensoren (1, 3) zeitgleich elektrische oder magnetische Aktivitäten des Gehirns (7) gemessen werden,
wobei mittels des Verfahrens nach einem der Ansprüche 1 bis 13 für jede paarweise Kombination der Sensoren (1, 3) ein paarspezifisches Maß für einen Zusammenhang zwischen den mit den beiden Sensoren (1, 3) gemessenen elektrischen oder magnetischen Aktivitäten des Gehirns (7) bestimmt wird und
die so gewonnene Vielzahl von paarspezifischen Maßen in einer Matrix angeordnet werden, die das Maß für einen Zusammenhang zwischen der Vielzahl von elektrischen oder magnetischen Aktivitäten des Gehirns (7) darstellt.

15. System zur Durchführung eines Verfahrens nach einem der vorhergehenden Ansprüche mit wenigstens einem ersten elektrischen oder magnetischen Sensor (1) und einem zweiten elektrischen oder magnetischen Sensor (3), die mit einem geeigneten Mittel mit einer Datenverarbeitungsanlage verbunden sind, die angepasst ist, um die Verfahrensschritte nach einem der vorhergehenden Ansprüche durchzuführen.

**Claims**

1. A method for determining a measure for a relationship between an electric or magnetic activity of the brain (7) measured with a first sensor (1) and an electric or magnetic activity of the brain (7) measured with a second sensor (3) spaced from the first sensor (1), wherein in the method
a first time-dependent signal (9) output by the first sensor (1) and a second time-dependent signal (11) output by the second sensor (3) are simultaneously recorded,
one or more frequency ranges of the first signal (1) and the second signal (3) are selected, which are identical for the two signals, und

in each selected frequency range a relationship between the first signal (1) and the second signal (3) is determined by means of the following steps:

- determining from the first signal (9) a first frequency-limited signal (13) limited to the respective frequency range and from the second signal (11) a second frequency-limited signal (15) limited to the respective frequency range,
- determining a first envelope (23) of the first frequency-limited signal (13),
- determining a second envelope (25) of a portion of the second frequency-limited signal (15), wherein the portion has a lower linear correlation with the first frequency-limited signal (13) than the second frequency-limited signal (15), and
- determining a measure for the relationship between the first envelope (23) and the second envelope (25) as the measure for a relationship between the electric or magnetic activity of the brain (7) measured with the first sensor (1) and the electric or magnetic activity of the brain (7) measured with the second sensor (3).

2. The method according to claim 1, wherein the determining of the first frequency-limited signal (13) for each frequency range comprises a Fourier transformation of the first signal (9) in a plurality of different time windows (33) for determining at least one first complex valued Fourier coefficient (35) for the respective frequency range in each time window (33), wherein the corresponding complex valued Fourier coefficients (35) form the first frequency-limited signal (13), and

wherein the determining of the second frequency-limited signal (15) for each frequency range comprises a Fourier transformation of the second signal (11) in the time windows (33) for determining at least one second complex valued Fourier coefficient (43) for the respective frequency range in each time window (33), wherein the corresponding complex valued Fourier coefficients (43) form the second frequency-limited signal (15).

3. The method according to claim 1 or 2, wherein the portion of the second frequency-limited signal (15) corresponds to the difference between the second frequency-limited signal (15) and the first frequency-limited signal (13) multiplied by a factor, wherein the factor describes a linear relationship between the first frequency-limited signal (13) and the second frequency-limited signal (15).

4. The method according to claim 1 or 2, wherein the portion of the second frequency-limited signal (15) corresponds to the portion of the second frequency-limited signal (15) the phase of which portion deviates from the phase of the first frequency-limited signal (13).

5. The method according to claim 4, wherein the portion of the second frequency-limited signal (15) is determined, the phase of which portion deviates by 90° from the phase of the first frequency-limited signal (13).

6. The method according to claim 2, wherein for each time window (33) the portion of the second frequency-limited signal (15) is determined as projection of the corresponding second frequency-limited signal (15) onto the direction in the complex plane (41), which direction is orthogonal to the corresponding first frequency-limited signal (13).

7. The method according to any of the preceding claims, **characterized in that** the determining of a relationship between the activity of the brain (7) measured with a first sensor (1) and with a second sensor (3) in each selected frequency range further comprises the following steps:

- determining a third envelope (49) of the second frequency-limited signal (15),
- determining a fourth envelope (51) of a portion of the first frequency-limited signal (13), wherein the portion has a lower linear correlation with the second frequency-limited signal (15) than the first frequency-limited signal (13),
- determining a measure for the relationship between the third envelope (49) and the fourth envelope (51), and
- determining a mean measure for the relationship between the electric or magnetic activity of the brain (7) measured with the first sensor (1) and the electric or magnetic activity of the brain (7) measured with the second sensor (3) from the measure for the relationship between the first envelope (23) and the second envelope (25) and from them measure for the relationship between the third envelope (49) and the fourth envelope (51).

8. The method according to claim 7, wherein the portion of the first frequency-limited signal (13) corresponds to the difference between the first frequency-limited signal (13) and the second frequency-limited signal (15) multiplied by a factor, wherein the factor describes a linear relationship between the second frequency-limited signal (15) and the first frequency-limited signal (13).

9. The method according to claim 7, wherein the portion of the first frequency-limited signal (13) corresponds to the portion of the first frequency-limited signal (13) the phase of which portion deviates from the phase of the second frequency-limited signal (15), wherein the deviation of the portion of the phase of the first frequency-limited signal (13) from the phase of the second frequency-limited signal (15) is preferably 90°.

10. The method according to claim 7, if dependent on claim 2, wherein for each time window (33) the portion of the first frequency-limited signal (13) is determined as projection of the corresponding first frequency-limited signal (13) onto the direction in the complex plane (41), which direction is orthogonal to the corresponding at least one second frequency-limited signal (15).

11. The method according to any of the preceding claims, wherein at the first sensor (1) and the second sensor (3) the electric activity of the brain (7) is measured by means of electroencephalography (EEG) or electrocorticogram (ECoG) or the magnetic activity of the brain (7) is measured by means of magnetoencephalography (MEG).

12. The method according to any of the preceding claims, **characterized in that** the first sensor (1) comprises a plurality of individual sensors, wherein the first time-dependent signal (9) is formed from a linear combination of the signals output by the individual sensors of the first sensor (1), and

that the second sensor (3) comprises a plurality of individual sensors, wherein the second time-dependent signal (11) is formed from a linear combination of the signals output by the individual sensors of the second sensor (3).

13. The method according to claim 12, wherein a signal of a first source is determined from the signals of the individual sensors of the first sensor (1) by means of source localization, which signal of a first source is output as first time-dependent signal (9) of the first sensor (1), and

wherein a signal of a second source is determined from the signals of the individual sensors of the second sensor (3) by means of source localization, which signal of a second source is output as second time-dependent signal (11) of the second sensor (3).

14. A method for determining a measure for a relationship between a plurality of electric or magnetic activities of the brain (7), wherein electric or magnetic activities of the brain (7) are simultaneously measured by means of a plurality of sensors (1, 3),

wherein for each pairwise combination of the sensors (1, 3) a pair-specific measure for a relationship between the electric or magnetic activities of the brain (7) measured with the two sensors (1, 3) is determined by means of the method according to any of claims 1 to 13, and

the plurality of pair-specific measures obtained in this manner is arranged in a matrix which represents the measure for a relationship between the plurality of electric or magnetic activities of the brain (7).

15. A system for carrying out a method according to any of the preceding claims, comprising at least one first electric or magnetic sensor (1) and a second electric or magnetic sensor (3), which are connected by means of a suitable means to a data processing system adapted to carry out the method steps according to any of the preceding claims.

**Revendications**

1. Procédé pour déterminer une mesure pour un rapport entre une activité électrique ou magnétique du cerveau (7) mesurée avec un premier capteur (1) et une activité électrique ou magnétique du cerveau (7) mesurée avec un second capteur (3) placé espacé du premier capteur (1), cependant que dans ce procédé

un premier signal dépendant du temps (9), émis par le premier capteur (1) et un second signal dépendant du temps (11) émis par le second capteur (3) sont enregistrés simultanément,

une ou plusieurs gammes de fréquences du premier signal (1) et du second signal (3) qui sont identiques pour les deux signaux sont sélectionnées et

dans chaque gamme de fréquences sélectionnées un rapport entre le premier signal (1) et le second signal (3) est déterminé avec les étapes suivantes :

- détermination d'un premier signal de fréquence limitée (13), limité à la gamme de fréquences respective, à partir du premier signal (9) et d'un second signal de fréquence limitée (15), limité à la gamme de fréquences respective, à partir du second signal (11),
- détermination d'une première enveloppe (23) du premier signal de fréquence limitée (13),
- détermination d'une seconde enveloppe (25) d'une fraction du second signal de fréquence limitée (15), cependant que la fraction présente une corrélation linéaire plus faible avec le premier signal de fréquence limitée (13) que le second signal de fréquence limitée (15) et
- détermination d'une mesure pour le rapport entre la première enveloppe (23) et la seconde enveloppe (25) comme mesure pour un rapport entre l'activité électrique ou magnétique du cerveau (7) mesurée avec le premier capteur (1) et l'activité électrique ou magnétique du cerveau (7) mesurée avec le second capteur (3).

2. Procédé selon la revendication 1 pour lequel la détermination du premier signal de fréquence limitée (13) comprend, pour chaque gamme de fréquences, une transformation de Fourier du premier signal (9) dans une multitude de différentes fenêtres de temps (33) pour déterminer au moins un premier coefficient de Fourier de valeur complexe (35) pour la gamme de fréquences respective dans chaque fenêtre de temps (33), cependant que les coefficients de Fourier de valeur complexe correspondants (35) forment le premier signal de fréquence limitée (13) et pour lequel la détermination du second signal de fréquence limitée (15) comprend, pour chaque gamme de fréquences, une transformation de Fourier du second signal (11) dans les fenêtres de temps (33) pour déterminer un second coefficient de Fourier de valeur complexe (43) pour la gamme de fréquences respective dans chaque fenêtre de temps (33), cependant que les coefficients de Fourier de valeur complexe correspondants (35) forment le second signal de fréquence limitée (15).

3. Procédé selon la revendication 1 ou 2 pour lequel la fraction du second signal de fréquence limitée (15) correspond à la différence entre le second signal de fréquence limitée (15) et le premier signal de fréquence limitée (13) multiplié avec un facteur, cependant que le facteur décrit un rapport linéaire entre le premier signal de fréquence limitée (13) et le second signal de fréquence limitée (15).

4. Procédé selon la revendication 1 ou 2 pour lequel la fraction du second signal de fréquence limitée (15) correspond à la fraction du second signal de fréquence limitée (15) dont la phase diffère de la phase du premier signal de fréquence limitée (13).

5. Procédé selon la revendication 4 pour lequel c'est la fraction du second signal de fréquence limitée (15) dont la phase diffère de 90° de la phase du premier signal de fréquence limitée (13) qui est déterminée.

6. Procédé selon la revendication 2 pour lequel pour chaque fenêtre de temps (33) la fraction du second signal de fréquence limitée (15) est déterminée comme projection du second signal de fréquence limitée correspondant (15) sur la direction orthogonale au premier signal de fréquence limitée correspondant (13) dans le plan complexe (41).

7. Procédé selon l'une des revendications précédentes, **caractérisé en ce que** la détermination d'un rapport entre l'activité du cerveau (7) mesurée avec un premier capteur (1) et avec un second capteur (3) dans chaque gamme de fréquences comprend de plus les étapes suivantes :

   - détermination d'une troisième enveloppe (49) du second signal de fréquence limitée (15),
   - détermination d'une quatrième enveloppe (51) d'une fraction du premier signal de fréquence limitée (13), cependant que la fraction présente une corrélation linéaire plus faible avec le second signal de fréquence limitée (15) que le premier signal de fréquence limitée (13),
   - détermination d'une mesure pour le rapport entre la troisième enveloppe (49) et la quatrième enveloppe (51) et
   - détermination d'une mesure moyenne pour le rapport entre l'activité électrique ou magnétique du cerveau (7) mesurée avec le premier capteur (1) et l'activité électrique ou magnétique du cerveau (7) mesurée avec un second capteur (3) à partir de la mesure pour le rapport entre la première enveloppe (23) et la seconde enveloppe (25) et à partir de la mesure pour le rapport entre la troisième enveloppe (49) et la quatrième enveloppe (51).

8. Procédé selon la revendication 7 pour lequel la fraction du premier signal de fréquence limitée (13) correspond à la différence entre le premier signal de fréquence limitée (13) et le second signal de fréquence limitée (15) multiplié avec un facteur, cependant que le facteur décrit un rapport linéaire entre le second signal de fréquence limitée (15) et le premier signal de fréquence limitée (13).

9. Procédé selon la revendication 7 pour lequel la fraction du premier signal de fréquence limitée (13) correspond à la fraction du premier signal de fréquence limitée (13) dont la phase diffère de la phase du second signal de fréquence limitée (15), cependant que la divergence de la fraction de la phase du premier signal de fréquence limitée (13) par rapport à la phase du second signal de fréquence limitée (15) est de préférence de 90°.

10. Procédé selon la revendication 7, dans cette mesure dépendant de la revendication 2, pour lequel pour chaque fenêtre de temps (33) la fraction du premier signal de fréquence limitée (13) est déterminée comme projection du premier signal de fréquence limitée correspondant (13) sur la direction orthogonale au second signal de fréquence limitée correspondant qui existe au moins (15) dans le plan complexe (41).

11. Procédé selon l'une des revendications précédentes pour lequel l'activité électrique du cerveau (7) est mesurée sur le premier capteur (1) et sur le second capteur (3) par électroencéphalographie (EEG) ou électrocorticogramme (ECoG) ou l'activité magnétique du cerveau est mesurée par magnétoencéphalographie (MEG).

12. Procédé selon l'une des revendications précédentes, **caractérisé en ce que** le premier capteur (1) présente une multitude de capteurs individuels, cependant que le premier signal dépendant du temps (9) est formé à partir d'une combinaison linéaire des signaux émis par les capteurs individuels du premier capteur (1) et que le second capteur (3) présente une multitude de capteurs individuels, cependant que le second signal dépendant du temps (11) est formé à partir d'une combinaison linéaire des signaux émis par les capteurs individuels du second capteur.

13. Procédé selon la revendication 12, cependant qu'un signal d'une première source qui est émis comme premier signal dépendant du temps (9) du premier capteur (1) est déterminé à partir des signaux des capteurs individuels du premier capteur (1) par localisation de source et cependant qu'un signal d'une seconde source qui est émis comme second signal dépendant du temps (11) du second capteur (3) est déterminé à partir des signaux des capteurs individuels du second capteur (3) par localisation de source.

14. Procédé pour déterminer une mesure pour un rapport entre une multitude d'activités électriques ou magnétiques du cerveau (7) pour lequel des activités électriques ou magnétiques du cerveau (7) sont mesurées simultanément à l'aide d'une multitude de capteurs (1, 3), cependant qu'au moyen du procédé selon l'une des revendications 1 à 13 une mesure spécifique d'une paire est déterminée pour chaque combinaison par paires des capteurs (1, 3) pour un rapport entre les activités électriques ou magnétiques du cerveau (7) mesurées avec les deux capteurs (1, 3) et la multitude ainsi obtenue de mesures spécifiques à la paire est placée dans une matrice qui représente la mesure pour un rapport entre la multitude d'activités électriques ou magnétiques du cerveau (7).

15. Système pour exécuter un procédé selon l'une des revendications précédentes avec au moins un premier capteur électrique ou magnétique (1) et un second capteur électrique ou magnétique (3) qui sont reliés avec un moyen approprié à une installation de traitement de données qui est adaptée pour exécuter les étapes de procédé selon l'une des revendications précédentes.

**Fig. 1**

EP 2 793 693 B1

**Fig. 2**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 20010049480 A1

### In der Beschreibung aufgeführte Nicht-Patentliteratur

- **NOLTE et al.** Identifying true brain interaction from EEG data using the imaginary part of coherency. *Clinical Neurophysiology,* 2004, vol. 115, 2292-2307